# EUROPÄISCHE PATENTSCHRIFT

(11) **EP 4 363 803 B1**
(45) Veröffentlichungstag und Bekanntmachung des Hinweises auf die Patenterteilung: **22.10.2025**
(21) Anmeldenummer: 22743702.7
(22) Anmeldetag: 23.06.2022
(51) Int. Cl.: G01D 21/00

(54) **SENSORNETZ**
SENSOR NETWORK
RÉSEAU DE DÉTECTION

(30) Priorität: 28.06.2021 AT 1182021
(43) Veröffentlichungstag der Anmeldung: 08.05.2024
(73) Patentinhaber: Sendance GmbH, 4040 Linz (AT)
(72) Erfinder: KOEPPE, Robert, 4040 Linz (AT)
(74) Vertreter: Burgstaller, Peter
(86) Internationale Anmeldenummer: PCT/AT2022/060213
(87) Internationale Veröffentlichungsnummer: WO 2023/272319

(56) Entgegenhaltungen:
- WO-A1-2017/013493
- CN-A- 109 341 727

## Beschreibung

Die Erfindung betrifft ein flexibles Sensornetz.

Ein Sensornetz umfasst mehrere Einzelsensoren, welche an Knotenpunkten des Netzes vorliegen und durch die Netzstruktur miteinander verbunden sind.

Solche Sensorsetze können mit herkömmlichen gemantelten Leitern wie Litzenkabel realisiert werden, welche als Verbindungen zwischen den Sensoren des Netzes dienen. Die Sensoren können insofern flexibel an einer Oberfläche angeordnet werden, indem deren Abstand kleiner oder gleich der Länge der Litzenkabel sein kann. Nachteilig ist, dass ein Litzenkabel bei einem Abstand der Sensoren, welche kleiner seiner Länge ist, entweder von der Oberfläche absteht oder in einem gewundenen Verlauf verlegt werden muss.

Dieser Stand der Technik ist beispielsweise aus der CN109341727A bekannt, bei welcher die Verbindungselemente als isolierte Leiter ausgeführt sind und zur Gänze einen gewundenen Verlauf aufweisen.

Bei der EP3621088A1, der US2017303853A1, der US2018249767A1 und der WO2017013493A1 werden flexible Verbindungen genutzt um vordefinierte Schaltungen zu realisieren.

Bei der US2010238636A1 und US2020060558A1 werden Schaltungen zur Gänze in flexibles Material eingegossen.

Die der Erfindung zu Grunde liegende Aufgabe besteht darin, ein flexibles Sensornetz zu schaffen, welches einfach in der Herstellung ist und eine flexible Anbringung der einzelnen Sensoren an einer Oberfläche ermöglicht.

Für das Lösen der Aufgabe wird ein Sensornetz gemäß Anspruch 1 vorgeschlagen, sowie dessen Verwendung an einem Körper und Verfahren zur Durchführung von Messungen mit dem Sensornetz.

Insbesondere wird ein zweidimensionales Netz vorgeschlagen, umfassend Sensorpunkte, welche die Knotenpunkte des Netzes bilden und längliche Verbindungselemente, umfassend ein Substrat und einen Leiter, welche Verbindungselemente die Kanten des Netzes bilden, wobei das Netz in jeder seiner zwei Dimensionen zumindest zwei Kanten aufweist, welche Gitterlinien des Netzes bilden, wobei das Netz aus Sensorpunkten und Verbindungselementen im Bereich zwischen den Kanten offen ist, wobei an jedem Sensorpunkt ein Stromfluss oder eine kapazitive Kopplung von den Leitern einer ersten Gitterlinie zu den Leitern einer zweiten Gitterlinie möglich ist, wobei die erste und zweite Gitterlinie quer zueinander stehen und der Stromfluss oder die kapazitive Kopplung von einer Messgröße des Sensorpunkts abhängig ist, wobei die Verbindungselemente jeweils ein dehnbares Substrat aufweisen, welches Substrat als ein gerader Streifen vorliegt, auf welchem zumindest ein Leiter vorliegt, welcher von einem Endbereich des Verbindungselements entlang dessen Längsrichtung zu einem zweiten Endbereich des Verbindungselements verläuft, wobei der Leiter aus einem nicht dehnbaren Material besteht und der Leiter einen gewundenen oder geknickten Verlauf am dehnbaren Substrat aufweist, sodass die einzelnen Abschnitte des Leiters quer zur Längsrichtung des jeweiligen Verbindungselements verlaufen.

Das Sensornetz umfasst in einer Ausführungsvariante mehrere Sensorpunkte und mehrere dehnbare Verbindungselemente, welche jeweils zumindest einen Leiter umfassen, wobei die Sensorpunkte zur Ausbildung einer zweidimensionalen Gitterstruktur mit den dehnbaren Verbindungselementen verbunden sind.

Bevorzugt wird das Sensornetz hergestellt indem zuerst Sensorpunkte und Verbindungselemente hergestellt werden, insbesondere getrennt voneinander und nachfolgend die Sensorpunkte durch das Anbringen der Verbindungselemente zu einem Sensornetz verbunden werden. Danach kann das fertige Sensornetz an einen Körper angebracht werden.

Als Körper ist insbesondere ein physikalischer Körper (physical body or physical object) zu verstehen.

Das Netz kann auf ein in zwei Dimensionen gekrümmtes, frei geformtes Bauteil flächig aufgebracht werden, ohne dass die Verbindungslängen im Vorfeld speziell angepasst werden müssen. Durch die Netzstruktur müssen die Verbindungselemente nur in einer Dimension dehnbar sein. Mit einer einheitlichen Fertigungsmethode (Verknüpfen von Sensorpunkten) können verschiedene Flächen erzeugt werden. Somit kann ein Standard-Sensornetz in ein speziell angefertigtes Bauteil integriert werden. Beispiele hierfür sind speziell an den Träger angepasste Orthesen, Prothesenschäfte, Skischuhe, Schuheinlagen oder Sitzschalen.

Die Sensorpunkte weisen bevorzugt eine regelmäßige Form auf. Die Sensorpunkte können bevorzugt einen runden oder viereckigen, oder achteckigen Außenumfang aufweisen, oder viereckig mit abgerundeten oder abgeschrägten Ecken ausgeführt sein. Bevorzugt weist jeder Sensorpunkt vier Außenkanten auf, wobei gegenüberliegende Außenkanten bevorzugt parallel zueinander liegen. Bevorzugt sind die vier Außenkanten ident zueinander ausgeführt. Die Sensorpunkte können eine eindeutige Markierung, insbesondere in einem Eckbereich, oder eine eindeutige Form, beispielsweise durch das Abschrägen einer Ecke, aufweisen, sodass die Ausrichtung der Sensorpunkte in der Netzanordnung ersichtlich ist, insbesondere um Fehlmontagen zu verhindern.

Die Sensorpunkte weisen bevorzugt einen Durchmesser oder einen Abstand zwischen zwei gegenüberliegenden Kanten im Bereich von 3mm bis 3cm auf, besonders bevorzugt 5mm bis 2cm. Bevorzugt sind die Sensorpunkte kleinflächige, dünne Folien mit elektrisch aktiven Schichten. Die Sensorpunkte ändern bei äußeren Einwirkungen bevorzugt zumindest eine ihrer elektrischen Eigenschaften messbar.

Die Sensorpunkte liegen mit Abstand zueinander vor, bevorzugt in mehreren parallelen Reihen.

Die Sensorpunkte sind mit den jeweils direkt benachbarten Sensorpunkten des Netzes durch dehnbare Verbindungselemente verbunden, welche bevorzugt mindestens auf 125%, insbesondere mindestens 150%, besonders bevorzugt 200% der ungedehnten Länge dehnbar sind, ohne Änderung des Widerstands des elektrischen Leiters des Verbindungselements. Die Dehnbarkeit des Verbindungselements liegt bevorzugt im Bereich des 1,25 bis 3-fachen seiner ungedehnten Länge. Das Substrat der Verbindungselemente ist in diesem Bereich elastisch verformbar. Das Substrat kann somit zumindest auf das 1,25-fache, bevorzugt zumindest 1,5-fache, insbesondere zumindest doppelte seiner ungedehnten Länge elastisch verformt werden.

Der Abstand im ungedehnten Netz zwischen den Sensorpunkten, bzw. die Länge eines Verbindungselements zwischen zwei Sensorpunkten, beträgt bevorzugt zwischen 1cm und 4cm, insbesondere zwischen 1,5cm und 3cm. Durch die Dehnbarkeit der Verbindungselemente kann der Abstand der Sensorpunkte am Körper deutlich höher ausfallen, wobei die Sensorpunkte einen Abstand im Bereich von 1cm (ungedehnte "1cm"-Verbindungselemente) bis 12cm (zb. voll gedehnte 3-fach dehnbare "4cm"-Verbindungselemente) aufweisen können.

Das resultierende Netz ist bevorzugt auf einer frei geformten Oberfläche eines Körpers, im Sinne eines Gegenstandes bzw. Objekts, angebracht. Bevorzugt sind sowohl die Sensorpunkte als auch die Verbindungselemente flächig mit dem Körper verbunden, insbesondere auf diesen aufgeklebt, oder in dessen Schichtaufbau integriert. Die Sensorpunkte ändern bei äußeren Einwirkungen auf den Körper zumindest eine elektrische Eigenschaft messbar. Je nach Größe und Form der zu bedeckenden Oberfläche des Körpers kann eine unterschiedliche Anzahl von Sensorpunkten vorgesehen und als Netzstruktur verknüpft sein.

Damit das Netz aufklebbar ist, weist dieses bevorzugt eine Klebeschicht auf. Bevorzugt liegt die Klebeschicht einseitig an den Sensorpunkten und den Verbindungselementen vor. In einer anderen Ausführungsvariante sind nur die Sensorpunkte mit einer Klebeschicht versehen. In einer anderen Ausführungsvariante sind nur die Verbindungselemente mit einer Klebeschicht versehen.

In einer anderen Ausführungsvariante sind die Sensorpunkte und/oder die Verbindungselemente beidseits mit einer Klebeschicht versehen.

In einer Ausführungsvariante liegt an der Klebeschicht eine abziehbare Trennfolie vor.

Der Körper weist bevorzugt eine in zwei Dimensionen gekrümmte Oberfläche auf. Der Körper ist bevorzugt ein Gegenstand oder ein Teil eines Gegenstandes und nicht, wie weniger bevorzugt auch möglich, etwa der Körper oder ein Körperteil eines Menschen oder Tieres. Der Körper kann aber beispielsweise ein Baum insbesondere ein Baumstamm sein.

Der Körper umfasst bevorzugt Kunststoff, Schaumstoff oder Holz oder besteht zur Gänze aus einem oder mehreren dieser Materialien.

Der Körper, in Form eines Gegenstandes, weist in einer Ausführungsvariante Vertiefungen für die Sensorpunkte auf. Der Körper, in Form eines Gegenstandes, weist in einer Ausführungsvariante Vertiefungen für die Verbindungselemente auf. In einer anderen Ausführungsvariante weist der Körper Vertiefungen für die Sensorpunkte und die Verbindungselemente auf. Bevorzugt liegen die Oberflächen der die Vertiefungen umgebenden erhabenen Bereiche des Körpers plan mit den in den Vertiefungen eingesetzten Sensorpunkten und/oder Verbindungselementen. Bevorzugt weisen mehrere oder alle Vertiefungen für die Sensorpunkte einen Abstand zueinander auf, welcher größer ist als die ungedehnte Länge der Verbindungselemente des Sensornetzes. Anders gesagt liegen mehrere oder alle Verbindungselement im gedehnten Zustand am Körper vor, insbesondere in Vertiefungen des Körpers.

Die Herstellung des Körpers mit Sensornetz erfolgt dabei indem zuerst ein Körper mit Vertiefungen bereitgestellt wird und dann das Sensornetz in den Vertiefungen platziert wird.

Eine entsprechende Verwendung des gegenständlichen Sensornetz besteht darin, dass der Körper ein Gegenstand ist, wobei in einem ersten Schritt der Gegenstand mit Vertiefungen geschaffen wird oder mit solchen versehen wird, wobei nach diesem ersten Schritt am Gegenstand zueinander beabstandete Vertiefungen in Größe der einzelnen Sensorpunkte vorhanden sind, welche die Positionen der Sensorpunkte vorgeben und wobei nachfolgend im zweiten Schritt das Netz am Körper platziert wird, wobei dabei die Sensorpunkte des Netzes in den Vertiefungen des Körpers angeordnet werden.

Bevorzugt wird dabei, dass der Körper im ersten Schritt mit zusätzlichen Vertiefungen in Größe der Verbindungselemente geschaffen wird oder mit solchen versehen wird, welche zusätzlichen Vertiefungen für Verbindungselemente die Vertiefungen für Sensorpunkte verbinden und wobei nachfolgend im zweiten Schritt das Netz am Körper platziert wird, wobei dabei die Sensorpunkte des Netzes in den Vertiefungen für Sensorpunkte angeordnet werden und die Verbindungselemente in den zusätzlichen Vertiefungen für Verbindungselemente.

Bevorzugt wird, dass mehrere Abstände, welche zwischen je zwei Sensorpunkten vorliegen und welche durch die Vertiefungen vorgegeben sind, länger sind als die ungedehnte Länge des jeweiligen Verbindungselements, welches zwischen den jeweiligen zwei Sensorpunkten verläuft. In anderen Worten weicht die Anordnung der Vertiefungen am Körper von der ungedehnten Form des Sensornetzes ab, wobei das Sensornetz nur unter Dehnung mehrerer Verbindungselemente in den Vertiefungen am Körper platzierbar ist. Bevorzugt weist der Körper eine netzartige Struktur von Vertiefungen auf, wobei die netzartige Struktur unregelmäßig vorliegt, also mit variierenden Längen der Kanten der netzartigen Struktur. Bevorzugt liegt das ungedehnte Sensornetz als regelmäßiges Netz vor, also mit identer Länge aller Kanten des Netzes. Als eine Kante des Netzes ist eine Verbindung zwischen zwei Knoten des Netzes definiert.

In einer Ausführungsvariante wird über dem Sensornetz eine Schutzschicht angebracht, welche das Sensornetz am Körper fixiert und/oder gewünschte Oberflächeneigenschaften bereitstellt. Oberflächeneigenschaften können beispielsweise sein: Materialart, Rauheit, Saugfähigkeit. Die Schutzschicht kann über ein erhaben am Körper vorliegendes Sensornetz aufgebracht werden. Die Schutzschicht kann über ein in Vertiefungen des Körpers vorliegendes Sensornetz aufgebracht werden. Die Schutzschicht kann als Stoff, Folie oder aushärtende bzw. filmbildende Flüssigkeit aufgebracht werden. In einer Ausführungsvariante überdeckt die Schutzschicht die Verbindungselemente und die offenen Maschen des Netzes, wobei die Sensorpunkte frei liegen oder zumindest ein Bereich des jeweiligen Sensorpunktes frei liegt.

Das Trägermaterial der Sensorpunkte kann in einer Variante ein in zwei Dimensionen dehnbares Material sein. In einer anderen Variante ist das Trägermaterial nicht dehnbar.

Bevorzugt ist das Netz in eine kraftübertragende Deckschicht eingebettet (z.B. in weichen Lack oder in eine verklebte Textilschicht). Die Deckschicht kann vor oder nach dem Anbringen am Körper angebracht werden. Die Deckschicht kann dem Körper zugewandt oder abgewandt liegen.

Bevorzugt wird das Netz auf die Oberfläche des Körpers geklebt.

In einer Variante kann das Netz nach der Verwendung wieder vom Körper abgelöst werden.

Der Körper ist bevorzugt verformbar (intrinsisch weich oder thermoplastisch).

Die zweidimensionale Gitterstruktur ist bevorzugt ein rechteckiges, insbesondere quadratisches, Gitter.

Bevorzugt sind die Maschen des Netzes entsprechend Durchlässen im Körper angeordnet. So wird erreicht, dass Luft oder Flüssigkeit durch den Körper und das Netz gelangen können.

Bevorzugt werden vor dem Anbringen des Netzes an definierten Stellen des Körpers Markierungen angebracht, an welchen beim Anbringen des Netzes die Sensorpunkte befestigt werden. Dadurch wird die Position jedes Sensors vorgegeben.

Bei einer bevorzugten Verwendung des Sensornetzes, welches an der Oberfläche eines Körpers angebracht ist, werden äußere Einwirkungen auf den Körper erfasst, indem die elektrischen Eigenschaften jedes Sensorpunkts im Netz gemessen werden und dann die Messwerte mit bekannten Positionen der Sensoren am Körper verknüpft werden. Dazu ist bevorzugt die Form des Körpers und die Position der Markierungen des Körpers in einem virtuellen 3D-Modell des Körpers erfasst, wobei die Messwerte der Sensorpunkte mit den Positionsdaten des 3D-Modells verknüpft werden. Beispielsweise kann so die Druckbelastung an definierten Punkten des Körpers erfasst werden.

Bevorzugt werden die Messwerte der Sensorpunkte entsprechend einer Farbskala (z.B. grün-gelb-rot-Skala) am virtuellen 3D-Objekt des Körpers dargestellt.

Bevorzugt wird aus einer Vielzahl von zyklisch wiederholten Messungen ein Video erstellt, welches die zeitliche Veränderung der Messwerte der Sensorpunkte am virtuellen 3D-Objekt darstellt.

Bevorzugt werden viele solcher Messungen in einer Datenbank gespeichert und für Analysen verwendet (z.B. Ganganalyse, wenn Sensoren in einem Schuh oder einer Prothese angeordnet sind).

In einer Ausführungsvariante werden die Messwerte am realen Körper dargestellt, indem eine repräsentative Darstellung, wie eine Farbskala oder ein Helligkeitswert, auf die Position des jeweiligen Sensors projiziert wird. In einer anderen Ausführungsvariante können LEDs entsprechend der Anordnung der Sensorpunkte am Körper vorliegen, welche repräsentativ zu den Messwerten der Sensorpunkte angesteuert werden. Das Anbringen der LEDs kann mit einem zusätzlichen Netz erfolgen oder auf eine andere Weise.

Bevorzugt werden Messungen von vielen Sensornetzen an verschiedenen Körpern vorgenommen und gespeichert und für weitergehende Analysen verwendet (z.B. zur Auswertung von Eigenschaften von verschiedenen Arten von Körpern oder den Einwirkungen auf einen Körper in verschiedenen Umgebungen).

Die Erfindung umfasst ein Verfahren zum Durchführen von Messungen an einem Körper, wobei
- im ersten Schritt ein digitales Modell des Körpers erstellt wird,
- im zweiten Schritt Positionen von Messpunkten im digitalen Modell platziert werden,
- im dritten Schritt ein Sensornetz, welches Sensorpunkte und dehnbare Verbindungselemente zwischen den Sensorpunkten umfasst, am realen Körper angebracht wird, wobei die Sensorpunkte am realen Körper an den Positionen der Messpunkte im digitalen Modell angeordnet sind,
- im vierten Schritt zumindest eine Messung der Messwerte an den Sensorpunkten vorgenommen wird.

Die Erfindung umfasst ein Verfahren zum Durchführen von Messungen an einem Körper, wobei
- im ersten Schritt der Körper mit Markierungen für Messpunkte hergestellt wird, oder der Körper mit Markierungen für Messpunkte versehen wird,
- im zweiten Schritt ein Sensornetz, welches Sensorpunkte und dehnbare Verbindungselemente zwischen den Sensorpunkten umfasst, am Körper angebracht wird, wobei die Sensorpunkte am Körper an den Positionen der Markierungen für die Messpunkte angeordnet sind,
- im dritten Schritt zumindest eine Messung der Messwerte an den Sensorpunkten vorgenommen wird.

Im Fall des Herstellen eines Körpers ist der Körper als Gegenstand zu verstehen. Das Versehen eines Körpers mit Markierung ist bei Körpern allgemein möglich.

Die beiden Verfahren lassen sich vorteilhaft kombinieren, indem der erste Schritt des zweiten Verfahrens (Vorsehen von Markierungen am realen Körper entsprechend den digitalen Messpunkten) vor dem dritten Schritt des ersten Verfahrens ausgeführt wird.

Bei beiden Verfahren wird zum Durchführen von Messungen an einem Körper ein zweidimensionales Sensornetz verwendet, welches Sensorpunkte als Knoten des Netzes und dehnbare Verbindungselemente zwischen den Sensorpunkten als Kanten des Netzes umfasst und welches Netz in jeder seiner zwei Dimensionen zumindest zwei Kanten aufweist, welche Gitterlinien des Netzes bilden, wobei das Netz aus Sensorpunkten und Verbindungselementen im Bereich zwischen den Kanten offen ist und wobei die dehnbaren Verbindungselemente ein dehnbares Substrat und einen Leiter umfassen. Das Sensornetz wird unter individuell vornehmbarer Dehnung der einzelnen Verbindungselemente am realen Körper angebracht, wobei die Sensorpunkte am realen Körper an den Positionen der Messpunkte im digitalen Modell angeordnet werden und/oder an den Positionen der Markierungen am Körper.

In bevorzugten Varianten dieser Verfahren werden die gemessenen Messwerte an den Sensorpunkten durch eine Software mit den Messpunkten im digitalen Modell verknüpft. Insbesondere werden die realen Messwerte an den Positionen der Messpunkte im digitalen Modell in Echtzeit dargestellt und/oder die realen Messwerte im zeitlichen Verlauf zu den Messpunkten im digitalen Modell gespeichert. Dazu werden der Messwert, der Zeitstempel bzw. Zeitpunkt der Messung und die Positionsdaten des Messpunkts im digitalen Modell bzw. am Körper in einer Datenbank gespeichert.

Bevorzugt werden zudem die Positionsdaten des realen Körpers im Raum erfasst und/oder der Bewegungsablauf des realen Körpers im Raum erfasst. Dies erfolgt beispielsweise durch Videoaufnahmen, durch Positionsdetektoren im Raum, oder durch zusätzliche Bewegungssensoren am Körper. Bevorzugt werden unter Einbeziehung dieser Daten die realen Messwerte an den Positionen der Messpunkte in einem digitalen Bewegungsmodell in Echtzeit dargestellt und/oder die realen Messwerte im zeitlichen Verlauf zu den Messpunkten im digitalen Bewegungsmodell gespeichert. Dazu werden der Messwert, der Zeitstempel bzw. Zeitpunkt der Messung, die Positionsdaten des Messpunkts im digitalen Modell bzw. am Körper, und die momentane absolute und/oder relative Lage des jeweiligen Messpunktes im Raum in einer Datenbank gespeichert.

Der Messwert am jeweiligen Sensorpunkt ist von der zu messenden Messgröße abhängig. Die Messgröße verursacht daher eine Änderung zumindest einer elektrischen Eigenschaft eines sensitiven Materials oder sensitiven Elements des Sensorpunktes.

Die Änderung der elektrischen Eigenschaften der elektroaktiven Schicht bzw. des sensitiven Elements, wenn ein äußerer Einfluss auf den Körper einwirkt kann resistiv, piezoresistiv, piezoelektrisch oder kapazitiv sein oder eine Impedanzänderung. Das sensitive Element kann auch als Thermoelement (Thermocouple), als chemischer Sensor oder als fotosensitives Element, wie Fotoresistor oder Fotodiode, vorliegen.

Die Verschaltung der Sensorpunkte im Netz erfolgt bevorzugt als passiv oder aktiv Matrix-Verschaltung.

Das Abgreifen der Sensorsignale des Netzes erfolgt bevorzugt über Sammelleitungen, welche an zwei zueinander angrenzenden und quer zueinander stehenden Seiten des Netzes vorliegen. Jede Sammelleitung verläuft entlang einer Dimension des zweidimensionalen Netzes. Bevorzugt umfasst die Sammelleitung zumindest einen Sammelleiter je Linie von Sensorpunkten, welche entlang dieser Dimension und quer zu dieser vorliegt. Die Sammelleitungen sind bevorzugt entsprechend den Verbindungselementen ausgeführt, wobei die Sammelleitung ein dehnbares Substrat umfasst, in welchem mehrere elektrische Leiter aus nicht dehnbarem Material integriert sind.

Die Sammelleitungen führen bevorzugt zu einer Elektronik, welche die Messignale digitalisiert. Die Elektronik ist bevorzugt ebenfalls am Körper befestigt. Die Elektronik kann mit einer Datenverarbeitungsanlage verbunden sein, entweder durch Kabel oder durch eine drahtlose Datenübertragungsvorrichtung.

Die Gitterstruktur ist bevorzugt regelmäßig, sodass jedes Element bzw. jede Masche des Gitters, welches von vier Verbindungselementen umschlossen ist, im ungedehnten Zustand dieselbe Form und Größe aufweist.

Bevorzugt weisen alle Verbindungselemente des Netzes, welche jeweils zwei Sensorpunkte verbinden und parallel zueinander angeordnet sind, die gleiche ungedehnte Länge auf.

Bevorzugt weisen alle Verbindungselemente des Netzes, welche jeweils zwei Sensorpunkte verbinden, die gleiche ungedehnte Länge auf.

Die dehnbaren Verbindungselemente umfassen jeweils ein dehnbares Substrat und zumindest einen, bevorzugt exakt einen, Leiter aus nicht dehnbarem Material, wobei der Leiter nicht auf geradem Weg am Substrat verläuft, sodass die Länge des Leiters länger ist als die Länge des Verbindungselements. Der Leiter ist bevorzugt nicht redundant ausgeführt, sodass dieser exakt einen leitenden Pfad entlang des Verbindungselements bereitstellt.

Bevorzugt ist die Länge des Leiters an einem Stück Substrat um das 1,25-fache bis 4-fache länger als die ungedehnte Länge des Stücks Substrats, insbesondere um das 1,5-fache bis 3-fache, besonders bevorzugt zumindest 2-fache.

In einer Ausführungsvariante wird ein Verbindungselement hergestellt, indem zumindest ein langgezogener, nicht dehnbarer Metallkörper in eine gedehnte dehnbare Schicht integriert wird, wobei sich der Metallkörper nach dem Relaxieren der Schichten auffaltet bzw. mäanderförmig verformt.

In einer Ausführungsvariante wird ein Verbindungselement hergestellt, indem zumindest ein gefalteter bzw. mäandrierter nicht dehnbarer Metallkörper in eine ungedehnte dehnbare Schicht integriert wird.

Durch beide Varianten wird ein Verbindungselement erhalten, das dehnbar und leitfähig ist, wobei sich die Leitfähigkeit des Verbindungselementes beim Dehnen nicht ändert, da die Länge und der Querschnitt des Metallkörpers unverändert bleiben (Es ändert sich nur der Winkel zwischen den gefalteten bzw. mäandrierenden Abschnitten des Metallkörpers).

In einer Ausführungsvariante liegt der Leiter spiralförmig vor.

In einer Ausführungsvariante liegt der Leiter mäanderförmig vor.

In einer Ausführungsvariante liegt der Leiter mit einem Zickzack-Verlauf vor.

In einer Ausführungsvariante liegt der Leiter mit einem sinusartigen Verlauf vor.

Der Leiter ist bevorzugt im Substrat eingeschlossen, wobei an beiden Enden des dehnbaren Verbindungselements Anschlussstellen vorliegen. Bevorzugt liegt der Leiter zwischen zwei Folienschichten des Substrats vor.

Das Substrat ist bevorzugt eine Kunststofffolie. Der Kunststoff ist bevorzugt ein Elastomer.

Die Verbindungselemente liegen bevorzugt als flächige Elemente vor, wobei die Flächen der Verbindungselemente in Ebene der Flächen der Sensorpunkte liegen. Die Verbindungselemente liegen bevorzugt rechteckig vor, wobei die längere Seite des Rechteckes eine Kante des Netzes bildet und der Leiter in Längsrichtung des Rechteckes verläuft.

Die Sensorpunkte bzw. die Sensoren können in unterschiedlichsten Varianten vorliegen, wobei die Sensoren hierin beschriebene bevorzugte Merkmale aufweisen können. An einem Sensornetz können auch zueinander unterschiedliche Sensoren angebracht sein.

Jeder Sensorpunkt weist zumindest ein sensitives Element auf, wobei zumindest eine elektrische Eigenschaft (beispielsweise der Ohm'sche Widerstand) des sensitiven Elements von einer Messgröße abhängig ist.

Das sensitive Element liegt zwischen zwei Kontakten des Sensorpunktes, wobei einer dieser Kontakte mit Verbindungselementen einer ersten Gitterlinie des Netzes verbunden ist und der zweite dieser Kontakte mit Verbindungselementen einer zweiten Gitterlinie des Netzes verbunden ist, wobei die beiden Gitterlinien quer zueinander stehen und sich im Sensorpunkt kreuzen.

In einer Ausführungsvariante liegt an zumindest einem Sensorpunkt eine Diode vor, welche in Serie mit dem sensitiven Element des Sensorpunktes geschaltet ist. Dadurch ist der Sensorpunkt richtungsselektiv ansteuerbar. Durch eine in gleicher Richtung angeordnete Diode an jedem Sensorpunkt, wird Stromfluss in unerwünschten Richtungen im Netz unterbunden, sodass sogenannter Crosstalk zwischen den Sensorpunkten verhindert wird.

In einer Ausführungsvariante liegt an zumindest einem Sensorpunkt ein Transistor vor, welcher in Serie mit dem sensitiven Element des Sensorpunktes geschaltet ist, wobei am Steuereingang des Transistors eine zusätzliche Steuerleitung vorliegt. Für die Steuerleitung des Transistors liegt an einem Verbindungselement, welches am Sensorpunkt anschließt ein zusätzlicher Leiter vor oder es ist ein zusätzliches Verbindungselement für die Steuerleitung vorhanden. Dadurch ist der Sensorpunkt über die Steuerleitung des Transistors individuell ansteuerbar, indem der Transistor leitend oder nichtleitend geschaltet wird.

In einer Ausführungsvariante weist der Sensorpunkt einen planaren bzw. zweidimensionalen Aufbau auf, wobei der Stromfluss oder eine kapazitive Kopplung in einer Ebene erfolgt. Dies bedeutet, dass die Elemente des Sensorpunktes in Form von Leiterbahnen, sensitivem Element und optional Diode oder Transistor in der Ebene des Trägermaterials oder in einer Ebene am Trägermaterial des Sensorpunktes vorliegen und auch der Stromfluss oder eine kapazitive Kopplung in dieser Ebene erfolgt. Ein Beispiel für einen solchen Aufbau ist ein Sensor in Form einer Fingerelektrode mit ineinandergreifenden kammartigen Elektroden, welche in einer Ebene vorliegen.

In einer Ausführungsvariante weist der Sensorpunkt eine Sandwichkonfiguration auf, bei welcher das sensitive Element bzw. das sensitive Material zwischen zwei Elektroden vorliegt, wobei die flächige Ausdehnung der Elektroden parallel zur Ebene des Netzes vorliegen und der Stromfluss oder eine kapazitive Kopplung zwischen den Elektroden, durch das sensitive Material hindurch, senkrecht zur Ebene des Netzes erfolgt. Die Elektroden, welche Leiterbahnen darstellen, liegen somit in parallelen, senkrecht auf die Fläche des Sensorpunkts beabstandeten Ebenen vor. Diese Variante mit Stromfluss in mehreren Ebenen parallel zur Fläche des Sensorpunktes und Stromfluss oder kapazitive Kopplung senkrecht zwischen diesen Ebenen wird hierin als dreidimensionaler Aufbau des Sensorpunktes bezeichnet.

Bei dieser Ausführungsvariante liegt eine erste Leiterbahn oder ein erster Anschlusspunkt an der ersten Elektrode vor und ein zweiter Anschlusspunkt an der zweiten Elektrode. Die Verbindungselemente einer ersten Gitterlinie des Netzes sind mit der ersten Elektrode verbunden und die Verbindungselementen einer zweiten Gitterlinie des Netzes sind mit der zweiten Elektrode verbunden, wobei die beiden Gitterlinien quer zueinander stehen und sich im Sensorpunkt kreuzen. Die Verbindungselemente sind dabei an den beiden gegenüberliegen flächigen Seiten des Sensorpunktes angeschlossen.

Für die Sensorpunkte ist bevorzugt, dass diese vier Anschlusspunkte aufweisen, an welchen Verbindungselemente elektrisch leitend anbringbar sind. Bevorzugt liegt ein erstes Paar dieser Anschlusspunkte auf einer Linie, die parallel zur ersten Dimension des Netzes ausgerichtet ist. Bevorzugt liegt das zweite Paar dieser Anschlusspunkte auf einer Linie, die parallel zur zweiten Dimension des Netzes ausgerichtet ist.

Bevorzugt ist das jeweilige Paar von Anschlusspunkten am Sensorpunkt durch einen elektrischen Leiter bzw. eine ununterbrochene Leiterbahn verbunden.

Bevorzugt sind alle Anschlusspunkte, welche entlang einer Gitterlinie des Netzes liegen, durch Verbindungselemente elektrisch leitend verbunden.

Der Sensorpunkt umfasst in einer Ausführungsvariante ein nichtleitfähiges Trägermaterial, auf welchem zumindest eine Leiterbahn vorliegt.

Die Leiterbahn ist aus leitfähigem Material gebildet, welches auf das Trägermaterial aufgebracht ist.

Der Sensor umfasst bevorzugt ein nichtleitfähiges Trägermaterial, welches durch ein Umgebungsmedium durchdringbar ist. Das Trägermaterial liegt bevorzugt als dünne flächige Schicht, beispielsweise in Form eines Blattes oder eines Streifens, vor.

Als durchdringbares Trägermaterial ist ein Trägermaterial zu verstehen, welches Öffnungen aufweist, welche sich von einer Seite des Trägermaterials zur anderen erstrecken.

Beim durchdringbaren Trägermaterial kann es sich um eine Folie, ein Gewebe, ein Vlies, eine Fasermatte oder einen offenzelligen Schaumstoff oder Schwamm handeln. Das Material kann zunächst als dichte Schicht hergestellt werden und danach durch Perforationen zu einem durchdringbaren Trägermaterial gemacht werden. Beispielsweise kann eine Folie durch Perforation zu einem durchdringbaren Trägermaterial gemacht werden.

Das durchdringbare Trägermaterial kann insbesondere aus Papier, Stoff, Glasfasern, mineralischen Fasern oder nicht-leitfähigem Kunststoff gebildet sein.

Bevorzugt ist das durchdringbare Trägermaterial im Bereich der Leiterbahn weiterhin durchdringbar, was bedeutet, dass das leitfähige Material die Öffnungen des durchdringbaren Trägermaterials nicht verschließt.

Das leitfähige Material liegt zumindest an einer Seite des Trägermaterials vor.

Bevorzugt umhüllt das leitfähige Material im Bereich der Leiterbahnen das Material des Trägermaterials vollständig. Dies bedeutet, dass das Material der Leiterbahnen an beiden Seiten des Trägermaterials vorliegt, wobei das Material der Leiterbahnen an den beiden Seiten durch die Öffnungen des Trägermaterials hindurch miteinander in Verbindung steht.

Anders formuliert umhüllt das Material der Leiterbahn das Material des Trägermaterials bevorzugt vollständig, welches zwischen zwei aneinander angrenzenden Öffnungen des Trägermaterials vorliegt. Bevorzugt verbleiben dabei im Bereich der Leiterbahn weiterhin Öffnungen des Trägermaterials, welche nicht durch das Material der Leiterbahn verschlossen sind.

Die Sensoren des Netzes können verwendet werden zur Messung von Temperatur, Dichteänderungen, mechanischen Verformungen (Druck, Dehnung, Stauchung, Biegung), chemischen Zustandsänderungen (z.B. Aushärten von Klebstoffen), Nässe, Eindringen von Flüssigkeiten, pH-Wert, biologische Wachstumsvorgänge, Konzentration von Biomolekülen, Zerstörung, Rissbildung.

Eine Kontaktierung der Leiterbahnen am Trägermaterial kann durch Anlöten von elektrischen Leitungen direkt an der Leiterbahn erfolgen. Es kann eine Klemme beidseits des Trägermaterials an einer Leiterbahn platziert werden. Es kann ein leitfähiges Material an der Leiterbahn aufgeklebt werden.

Das Trägermaterial und/oder die Leiterbahnen können mit reaktiven Oberflächen versehen sein, um beispielsweise pH-Wert oder Licht messen zu können.

Durch zwei getrennte Elektroden in einer verschachtelten bzw. ineinandergreifenden Kammstruktur ihrer Leiterbahnen lassen sich Veränderungen in den elektrischen Eigenschaften des Umgebungsmediums, bzw. des Trägermaterials im Zwischenraum der Kammstruktur, mit hoher Empfindlichkeit erfassen.

Mit einfachen Leitern bzw. einzelnen Leiterbahnen mit Kontaktstellen an beiden Enden können Temperaturänderung und/oder Dehnung gemessen werden.

Mit zwei sich kreuzenden Leiterbahnen aus unterschiedlichen Metallen - z.B. Nickel-Chrom / Nickel (Typ K) - können Thermoelemente aufgebaut werden. Dabei wird ausgenutzt, dass zwei Leiterbahnen aus unterschiedlichen Metallen an der Berührungsfläche einen thermoelektrischen Effekt aufweisen.

Bevorzugt ist das Trägermaterial maximal 2000 µm dick, besonders bevorzugt maximal 500 µm, insbesondere maximal 50 µm.

Bevorzugt weist das Trägermaterial eine Porosität von mindestens 10 % auf, besonders bevorzugt mindestens 50 %, insbesondere mindestens 75 %.

Bevorzugt weist das Trägermaterial eine durchschnittliche Porengröße von mindestens 1 µm auf, besonders bevorzugt mindestens 10 µm, insbesondere mindestens 100 µm.

Bevorzugt weist der Sensorpunkt im Bereich der Leiterbahn bzw. des leitfähigen Materials eine durchschnittliche Porosität von mindestens der Hälfte der Porosität des Trägermaterials auf.

Bevorzugt weist der Sensorpunkt im Bereich der Leiterbahn bzw. des leitfähigen Materials eine durchschnittliche Porengröße von mindestens der Hälfte der Porengröße des Trägermaterials auf.

Beim Material der Leiterbahnen handelt es sich bevorzugt um ein leitfähiges Metall, insbesondere Aluminium, Kupfer, Silber oder Gold, wobei Kupfer besonders bevorzugt wird. Zudem können Carbon Black und leitfähige Polymere verwendet werden.

Bevorzugt liegt das Material der Leiterbahnen in einer Schichtstärke von maximal 30 % der durchschnittlichen Porengröße vor, besonders bevorzugt maximal 10 %, insbesondere maximal 1%.

In einer Ausführungsvariante kann die Porosität so gewählt werden, dass der Sensor weitgehend durchsichtig erscheint und sich somit gut in eine optisch ansprechende Umgebung einfügen kann.

Der durchschnittliche Transmissionsgrad des Sensorpunkts beträgt bevorzugt zumindest 10 %, insbesondere zumindest 20 %, besonders bevorzugt zumindest 50 %, insbesondere zumindest 75 %.

Die hohe Transmission wird bevorzugt durch die Porosität erreicht, was bedeutet, dass das Material (z.b. die Fasern) des Trägermaterials nicht durchsichtig ist und/oder das Material der Leiterbahnen nicht durchsichtig ist.

In einer Ausführungsvariante kann die Porosität des bereits mit leitfähigem Material versehenen Trägermaterials oder des bereits mit Leiterbahnen versehenen Sensorpunkts vergrößert werden, indem dieses perforiert wird. Das Perforieren kann mechanisch oder durch Laser- oder Elektroperforation erfolgen. Dieses Perforieren kann im Bereich der Leiterbahnen und/oder im Bereich zwischen den Leiterbahnen erfolgen.

Die Erfindung wird anhand von Zeichnungen veranschaulicht:
- Fig. 1:: Veranschaulicht schematisch eine Ausführungsvariante eines Sensorpunktes mit zwei Verbindungselementen.
- Fig. 2:: Veranschaulicht schematisch die Verbindung von mehreren Sensorpunkten durch Verbindungselemente zur Ausbildung eines Netztes.
- Fig. 3:: Veranschaulicht eine beispielhafte Verwendung eines Sensornetzes an einem Körper.
- Fig. 4:: Zeigt eine Ausführungsvariante eines Sensorpunktes mit einer Diode.
- Fig. 5:: Zeigt eine Ausführungsvariante eines Sensorpunktes mit einem Transistor.
- Fig. 6:: Zeigt eine zweite Ausführungsvariante eines Sensorpunktes mit einem Transistor
- Fig. 7:: Zeigt eine Ausführungsvariante eines Sensorpunktes mit einem adressierbaren Schalter.
- Fig. 8:: Zeigt ein Netz mit aktiver Matrixschaltung.
- Fig. 9:: Zeigt ein Netz mit adressierbaren Sensorpunkten.
- Fig. 10:: Zeigt Sensorpunkte mit Elektroden im Sandwich-Aufbau.
- Fig. 11:: Zeigt eine Variante mit Fingerelektroden.
- Fig. 12:: Zeigt eine Variante von Sensorpunkten und Verbindungelementen.
- Fig. 13:: Zeigt eine erste Variante von Verbindungelementen in Form von Verbindungslinien.
- Fig. 14:: Zeigt eine zweite Variante von Verbindungslinien.
- Fig. 15:: Veranschaulicht beispielhafte Aufbauvarianten von Verbindungselementen.
- Fig. 16:: Veranschaulicht eine Aufbauvarianten einer Verbindungslinie.
- Fig. 17:: Veranschaulicht eine weitere Aufbauvariante einer Verbindungslinie.
- Fig. 18:: Veranschaulicht eine weitere Aufbauvariante einer Verbindungslinie.

Die in den Figuren gezeigten Ausführungsformen zeigen lediglich mögliche Ausführungsformen, wobei an dieser Stelle bemerkt sei, dass die Erfindung nicht auf diese speziell dargestellten Ausführungsvarianten derselben eingeschränkt ist, sondern auch Kombinationen der einzelnen Ausführungsvarianten untereinander und eine Kombination einer Ausführungsform mit der oben angeführten allgemeinen Beschreibung möglich sind. Diese weiteren möglichen Kombinationen müssen nicht explizit erwähnt sein, da diese weiteren möglichen Kombinationen aufgrund der Lehre zum technischen Handeln durch die gegenständliche Erfindung im Können des auf diesem technischen Gebiet tätigen Fachmannes liegen.

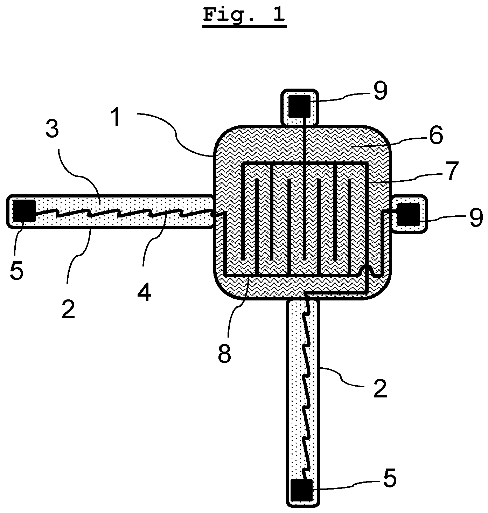

In Fig. 1 ist der grundlegende Aufbau der Sensorpunkte 1 und Verbindungselemente 2 anhand eines Beispiels veranschaulicht.

Die Verbindungselemente 2 weisen ein dehnbares Substrat 3 auf. Am oder im dehnbaren Substrat 3 liegt ein Leiter 4 aus nicht dehnbarem Metall vor. Der Leiter 4 verläuft in einem gewundenen oder geknickten Verlauf, sodass die einzelnen Abschnitte des Leiters 4 quer bzw. nicht parallel zur Längsrichtung der Verbindungselemente 2 verlaufen. Bei Längenänderung des Substrats 3 ändert sich der Winkel der einzelnen Abschnitte des Leiters 4 zur Längsrichtung, nicht aber der Querschnitt und die Länge des Leiters 4. Dadurch bleibt der elektrische Widerstand des Leiters 4 bei Längenänderung des Substrats 3 konstant.

Der Leiter 4 ist an beiden Enden des Verbindungselements 2 kontaktierbar. Dazu kann der Leiter 4 selbst am Substrat 3 freiliegen oder das Substrat 3 in Längsrichtung überragen. Zwischen den Kontaktstellen an den beiden Enden des Verbindungselements 2 ist der Leiter 4 bevorzugt isoliert, indem dieser selbst eine Isolierung aufweist oder insbesondere bevorzugt in das Substrat 3 eingebettet ist. Beispielsweise kann der Leiter 4 zwischen zwei Schichten des Substrats 3 in Form von Folien vorliegen.

Der Leiter 4 ist bevorzugt eine Einzellitze, kann aber auch mehrere Litzen umfassen. Das Material des Leiters 4 ist bevorzugt Kupfer. Der Leiter 4 wird bevorzugt spiralförmig verdreht und plattgedrückt. Die Spirale wird plattgedrückt bzw. in eine zweidimensionale Form gebracht. Alternativ kann der Leiter 4 durch Biegen oder Falten in eine zickzackförmige oder mäanderförmige oder sinusartige zweidimensionale Form gebracht werden.

Die zweidimensionale Struktur des umgeformten Leiters 4 kann dann gedehnt werden und im gedehnten Zustand auf ein gedehntes Substrat 3 aufgebracht werden. Alternativ kann die zweidimensionale Struktur ungedehnt oder auch gestaucht auf ein ungedehntes Substrat 3 aufgebracht werden.

Eine oder beide der Kontaktstellen am Verbindungselement 2 können als Anschlusspunkt 5 vorliegen, welcher in Form einer zweidimensionalen Fläche am Substrat 3 vorliegt, deren beide Dimensionen jeweils die Dicke des Leiters 4 übersteigen. Dadurch wird das Kontaktieren, insbesondere Verlöten, erleichtert.

Die Sensorpunkte 1 weisen ein Trägermaterial 6 auf, auf welchem Trägermaterial 6 zumindest eine Leiterbahn 7 verläuft. Im Fall von nur einer Leiterbahn 7 ist die Leiterbahn 7 selbst das sensitive Element des Sensorpunktes 1, wobei ein Ende der Leiterbahn mit einem in einer ersten Richtung vom Sensorpunkt 1 wegverlaufenden Verbindungselement 2 kontaktiert ist und das zweite Ende der Leiterbahn mit einem in einer zweiten Richtung vom Sensorpunkt 1 wegverlaufenden Verbindungselement 2 kontaktiert ist, wobei die erste und zweite Richtung quer zueinander stehen, insbesondere in einem Winkel von 90 Grad.

Bevorzugt verlaufen jedoch am Sensorpunkt 1 zumindest zwei Leiterbahnen 7, 8 welche nicht in direktem Kontakt stehen. Zwischen den Leiterbahnen 7, 8 liegt ein sensitives Material oder ein sensitives Element vor, welches einen Stromfluss von der ersten Leiterbahn 7 zur zweiten Leiterbahn 8 oder eine kapazitive Kopplung zwischen den Leiterbahnen 7 und 8 in Abhängigkeit einer von außen einwirkenden Eingangsgröße ermöglicht.

Die erste Leiterbahn 7 ist entweder mit einem Verbindungselement 2 kontaktiert, welches in eine erste Richtung verläuft. Oder die erste Leiterbahn 7 ist mit zwei Verbindungselementen 2 kontaktiert, welche in der ersten Richtung verlaufen. Die zweite Leiterbahn 8 ist entweder mit einem Verbindungselement 2 kontaktiert, welches in eine zweite Richtung verläuft. Oder die zweite Leiterbahn 8 ist mit zwei Verbindungselementen 2 kontaktiert, welche in der zweiten Richtung verlaufen. Die erste und die zweite Richtung stehen quer zueinander, insbesondere in einem Winkel von 90 Grad.

Der Sensorpunkt 1 weist bevorzugt zumindest einen Anschlusspunkt 9 für jede der beiden Richtungen auf, welcher in Form einer zweidimensionalen Fläche am Trägermaterial 6 vorliegt, deren beide Dimensionen jeweils die Breite der Leiterbahn 7 übersteigen. In einer Ausführungsvariante sind für jede der beiden Richtungen zwei Anschlusspunkte 9 vorhanden.

Bevorzugt liegt jeder Anschlusspunkt 9 mittig an der jeweiligen Seitenkante des Trägermaterials 6 vor.

Der Anschlusspunkt 9 kann sich am Trägermaterial 6 befinden oder wie dargestellt als zusätzliches Element anschließend an das Trägermaterial 6 vorliegen.

In der Ausführungsvariante der Fig. 1 ist das sensitive Element des Sensorpunkts 1 das Trägermaterial 6 selbst, welches zwischen den Leiterbahnen 7 und 8 vorliegt. Durch die Kammstruktur der ineinander verschachtelten Leiterbahnen 7, 8 wird die Sensitivität erhöht. Die horizontal auf einer Linie liegenden Anschlusspunkte 5 und 9 sind durch die Leiterbahn 8 und den Leiter 4 des Verbindungselements 2 direkt verbunden, sodass an diesen Anschlusspunkten dasselbe elektrische Potenzial vorliegt. Die vertikal auf einer Linie liegenden Anschlusspunkte 5 und 9 sind durch die Leiterbahn 7 und den Leiter 4 des Verbindungselements 2 direkt verbunden, sodass an diesen Anschlusspunkten dasselbe elektrische Potenzial vorliegt.

Wenn an einen der beiden Leiterbahnen 7, 8 eine Spannung angelegt wird, resultiert ein Stromfluss durch das Trägermaterial 6 hin zur anderen Leiterbahn 7, 8, sodass die Spannung an der zweiten der beiden Leiterbahnen 7, 8 ein Maß für die einwirkende Größe (wie beispielsweise Feuchtigkeit) auf das Trägermaterial 6 ist.

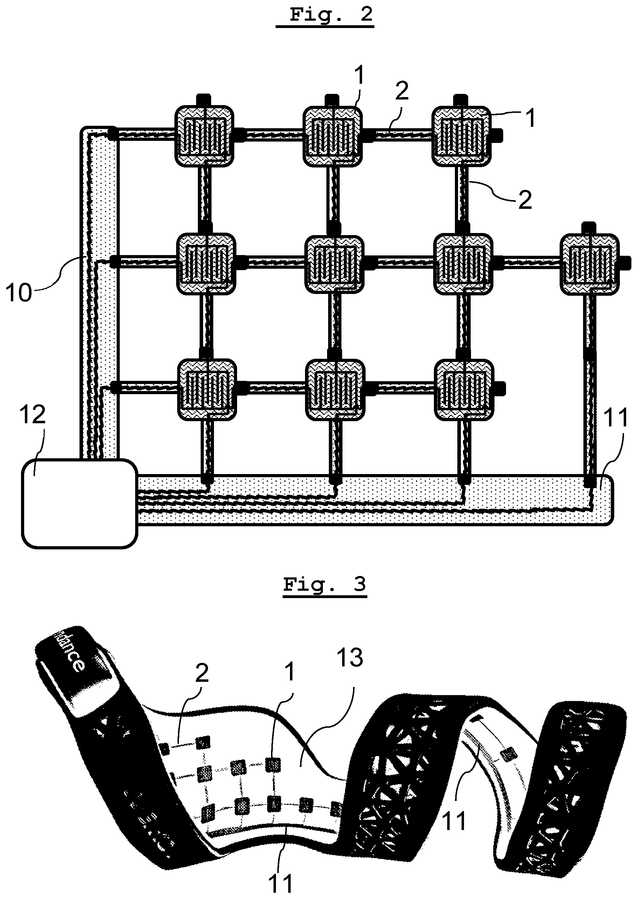

In Fig. 2 ist veranschaulicht, wie mehrere Sensorpunkte 1 und Verbindungselemente 2 zu einem Netz anordenbar sind und wie eine Messschaltung für das Netz realisierbar ist.

Die Sensorpunkte 1 bilden die Knotenpunkte des Netzes, welche durch die Verbindungselemente 2 (Kanten des Netzes) auf geradem Weg verbunden sind. Vier Verbindungselemente 2 bilden eine Masche, wobei das Netz im Raum innerhalb der Maschen offen ist.

Das Netz weist Gitterlinien auf, welche in zwei quer zueinander liegenden Richtungen verlaufen.

Alle Sensorpunkte 1, welche an einer Gitterlinie vorliegen, sind entlang der Gitterlinie durch Verbindungselemente 2 verbunden. Über Sammelleitungen 10, 11 werden die Gitterlinien mit einer Auswerteelektronik 12 verbunden, wobei je Gitterlinie ein Sammelleiter an einer der Sammelleitung 10, 11 von der jeweiligen Gitterline zur Auswerteelektronik 12 verläuft (bei einer passiven Matrix).

Bevorzugt sind die Sammelleiter der Sammelleitungen 10, 11 entsprechend den Leitern 4 der Verbindungselemente 2 ausgeführt. Bevorzugt ist das Substrat der Sammelleitungen 10, 11 gemäß dem Substrat 3 der Verbindungselemente 2 ausgeführt.

Beim Messvorgang wird immer an einem der Sammelleiter nach dem anderen einer der Sammelleitungen 10 eine elektrische Energie bzw. eine Spannung oder ein Signal angelegt, um immer nur eine Gitterlinie einer ersten Richtung des Netzes aktiv zu schalten. Die anderen Gitterlinien derselben Richtung werden bevorzugt auf Masse (Ground, GND) gelegt. An den Sammelleitern der anderen Sammelleitung 11 liegt jeweils das Messsignal jenes Sensorpunktes 1 an, der am Kreuzungspunkt der Gitterlinie des jeweiligen Sammelleiters und der aktiven Gitterlinie der ersten Richtung liegt.

Fig. 3 veranschaulicht die Anbringung eines Sensornetzes an einem Körper 13. Die Position der Sensorpunkte 1 am Körper 13 sind vorbestimmt und bevorzugt am Körper 13 markiert.

Durch Dehnen der Verbindungselemente 2 kann das Netz an unterschiedliche Distanzen zwischen parallelen Gitterlinien (insbesondere Zeilen und Spalten) angepasst werden.

Zudem kann sich der Körper selbst während der Messung dehnen und verformen, ohne dass das Messergebnis durch Längenänderung der Leiter 4 beeinflusst wird oder das Netz durch Bruch der Leiter 4 zerstört wird.

Der Körper 13 der Fig. 3 ist beispielsweise ein Kleidungsstück oder Stützelement aus Kunststoff und/oder Stoff, welches an einem Körperteil einer Person oder eines Tieres anbringbar ist.

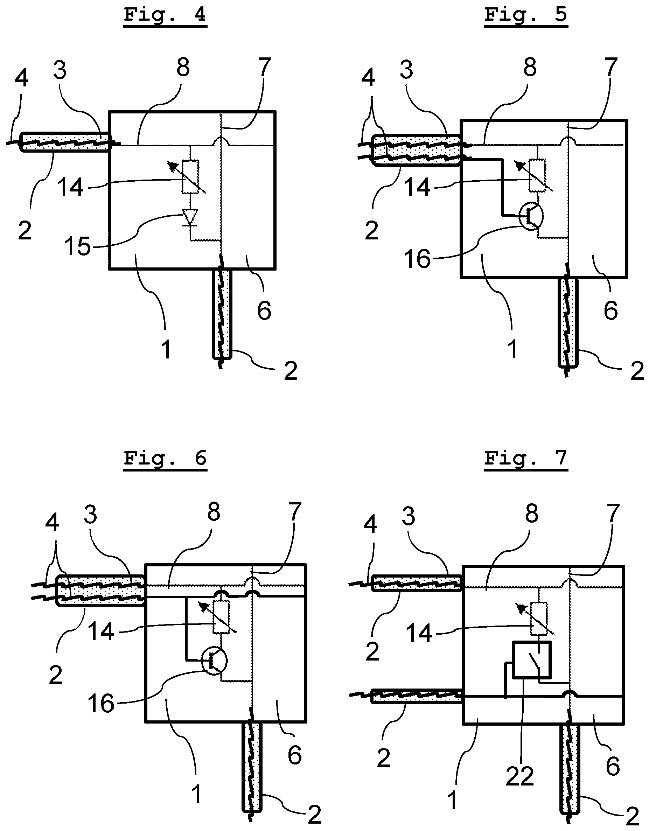

In Fig. 4 ist ein Sensorpunkt 1 veranschaulicht, welcher ein sensitives Element 14, beispielsweise in Form eines durch eine Messgröße veränderlichen Ohm'schen Widerstands, aufweist und in Serie zu diesem eine Diode 15 aufweist. Ein Stromfluss ist von der Leiterbahn 8 zur Leiterbahn 7 über das sensitive Element 14 und die Diode 15 in Durchlassrichtung möglich. Ein Stromfluss von Leiterbahn 7 zu Leiterbahn 8 hingegen wird durch die Diode 15 gesperrt. In einer Ausführungsvariante weist jeder Sensorpunkt 1 des Netzes eine Diode 15 auf, welche den Stromfluss von den Gitterlinien der ersten Richtung zu den Gitterlinien der zweiten Richtung unterbindet.

In Fig. 5 ist ein Sensorpunkt 1 veranschaulicht, welcher ein sensitives Element 14, beispielsweise in Form eines durch eine Messgröße veränderlichen Ohm'schen Widerstands, aufweist und in Serie zu diesem einen Transistor 16. Ein Stromfluss ist beispielsweise bei Verwendung eines npn-Transistors von der Leiterbahn 8 zur Leiterbahn 7 über den Transistor 16 möglich, wenn an dessen Basis bzw. Schalteingang eine Spannung anliegt. Allgemein formuliert ist bei dieser Variante eine beliebige Art von Transistor 16 am Sensorpunkt 1 angebracht, um den Strompfad zwischen den quer zueinander liegenden Verbindungselementen 2 am Sensorpunkt schaltbar zu machen. Bei Anordnung der Sensorpunkte 1 der Fig. 5 zu einem Netz kann jeder Transistor 16 mit einer eigenen Steuerleitung versehen sein, um jeden einzelnen Sensorpunkt einzeln schaltbar zu machen.

In der Variante der Fig. 6 liegen am Sensorpunkt 1 an zwei gegenüberliegenden Kanten des Sensorpunktes 1 Anschlussstellen für die Steuerleitung vor, um die Steuerleitungen der Sensorpunkte entlang einer Gitterlinie verbinden zu können.

In Fig. 7 ist ein adressierbarer Schalter 22 am Sensorpunkt angeschlossen. Die Steuerleitung ist in diesem Fall eine digitale Datenleitung. Dadurch kann jeder einzelne Sensorpunkt 1 einzeln geschaltet werden, auch wenn diese an einer gemeinsamen Steuerleitung vorliegen.

Wie in Fig. 5 und 6 veranschaulicht, können ein Leiter 4 für die Steuerleitung und ein Leiter 4 für eine der Leiterbahnen 7, 8 an einem gemeinsamen Verbindungselement 2 vorliegen.

Wie in Fig. 7 veranschaulicht, kann für die Steuerleitung ein eigenes Verbindungselement 2 mit einem einzelnen Leiter 4 am Sensorpunkt 1 angeschlossen sein.

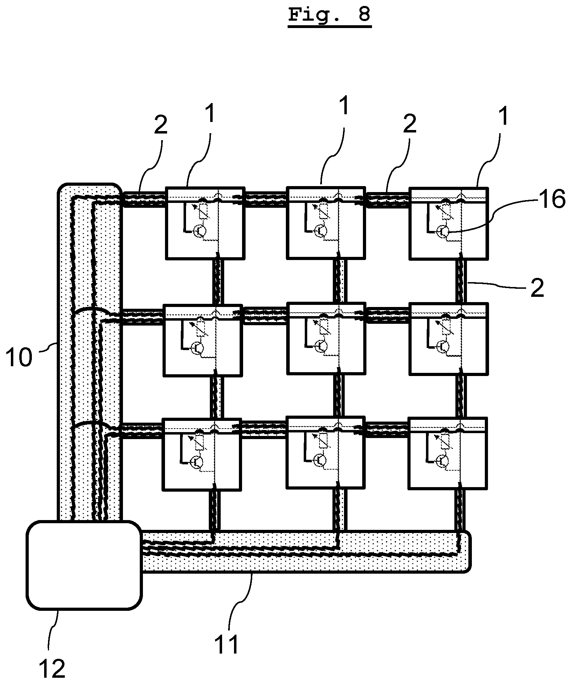

In Fig. 8 ist ein Netz mit Sensorpunkten 1 mit Transistoren 16 veranschaulicht. Die Steuerleitungen der Sensorpunkte sind entlang der Gitterlinien einer ersten Richtung des Netzes miteinander verbunden. Die Leiterbahnen 7, 8 der Sensorpunkte 1, welche in dieser ersten Richtung verbunden sind, können alle an einem gemeinsamen Potenzial, bzw. an einem Sammelleiter, liegen, da das Schalten der einzelnen Gitterlinien beim Messvorgang durch die Transistoren 16 über deren gemeinsame Steuerleitungen erfolgt, indem beim Messvorgang eine Steuerleitung nach der anderen aktiv geschaltet wird.

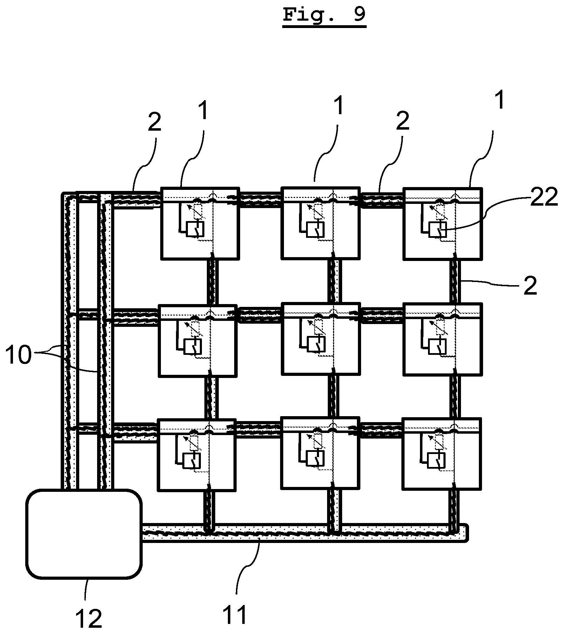

In Fig. 9 ist ein Netz mit Sensorpunkten 1 mit adressierbaren Schaltern 22 veranschaulicht. Die Steuerleitungen aller Sensorpunkte 1 sind miteinander verbunden, wobei dies durch Verbindungselemente 2 entlang der Gitterlinien einer ersten Richtung des Netzes erfolgen kann, wobei diese alle an einem gemeinsamen Sammelleiter angeschlossen sein können. Die Sensorpunkte liegen somit an einem Bus mit Bustopologie vor.

Die Leiterbahnen der Sensorpunkte, welche in einer gemeinsamen Richtung des Netzes verbunden sind, können alle an einem gemeinsamen Potenzial, bzw. an einem Sammelleiter, liegen, da das Schalten von einzelnen Sensorpunkten beim Messvorgang durch deren Adresse über den Bus erfolgen kann.

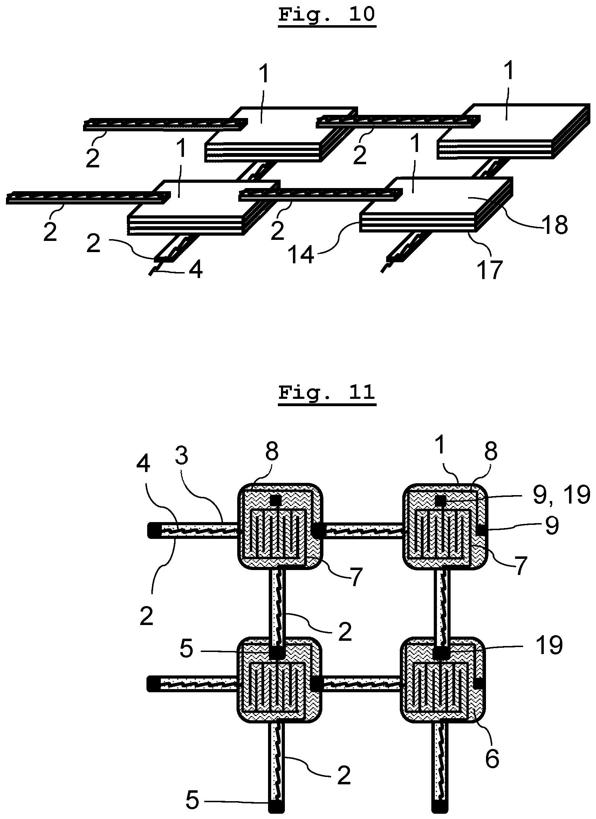

In Fig. 10 sind Sensorpunkte 1 mit Sandwich-Aufbau schematisch veranschaulicht. Die Sensorpunkte 1 weisen eine erste Elektrode 17 auf, welche der ersten Leiterbahn 7 der vorigen Ausführungsvarianten entspricht und eine zweite Elektrode 18, welche der zweiten Leiterbahn 8 der vorigen Ausführungsvarianten entspricht. Die Elektroden 17, 18 weisen jeweils einen zweidimensionalen flächigen Aufbau auf, welcher sich in der Ebene bzw. parallel zur Ebene des Netzes erstreckt. Die Elektroden 17, 18 können sich über die gesamte Fläche des Sensorpunktes 1 erstrecken oder nur über eine Teilfläche. Zwischen den Elektroden 17, 18 liegt das sensitive Element 14 bzw. ein sensitives Material vor, welches zumindest eine elektrische Eigenschaft in Abhängigkeit einer Messgröße ändert.

Die Verschaltung im Netz erfolgt dadurch, dass die ersten Elektroden 17 entlang je einer Gitterlinie einer ersten Richtung durch Verbindungselemente 2 verbunden sind und die zweiten Elektroden 18 entlang je einer Gitterlinie einer zweiten Richtung durch Verbindungselemente 2 verbunden sind, wobei die beiden Richtungen quer, insbesondere rechtwinkelig, zueinander stehen. Vorteilhaft ist, dass die Leiterbahnen 7, 8 bzw. die Elektroden 17, 18 in unterschiedlichen Ebenen vorliegen. Andernfalls, wenn Kreuzungspunkte der Leiterbahnen 7, 8 des Sensors in einer Ebene liegen, so wie in Fig. 1 im unteren, rechten Eckbereich des Sensorpunkts 1, dann muss an diesem Punkt eine isolierende Zwischenschicht zwischen den Leiterbahnen 7, 8 vorhanden sein. Der Sensorpunkt 1 der Fig. 10 weist einen "dreidimensionalen" Aufbau auf.

In Fig. 11 ist eine abgewandelte Form der Ausführungsvariante der Fig. 1 veranschaulicht, bei welcher Kreuzungen der Leiterbahnen 7, 8 am "zweidimensionalen" Sensorpunkt 1 vermieden werden. Dies erfolgt dadurch, dass eine Leiterbahn 8 außen um den Anschlusspunkt 9 der anderen Leiterbahn 7 herumgeführt wird. Die Leiterbahn 7 weist somit einen innenliegenden Anschlusspunkt 19 auf. Beim Anbringen eines Verbindungselements 2 am innenliegenden Anschlusspunkt 19 kreuzt der Leiter 4 des Verbindungselements 2, die Leiterbahn 8, wobei zwischen Leiter 4 und Leiterbahn 8 das elektrisch isolierende Substrat 3 des Verbindungselements 2 vorliegt.

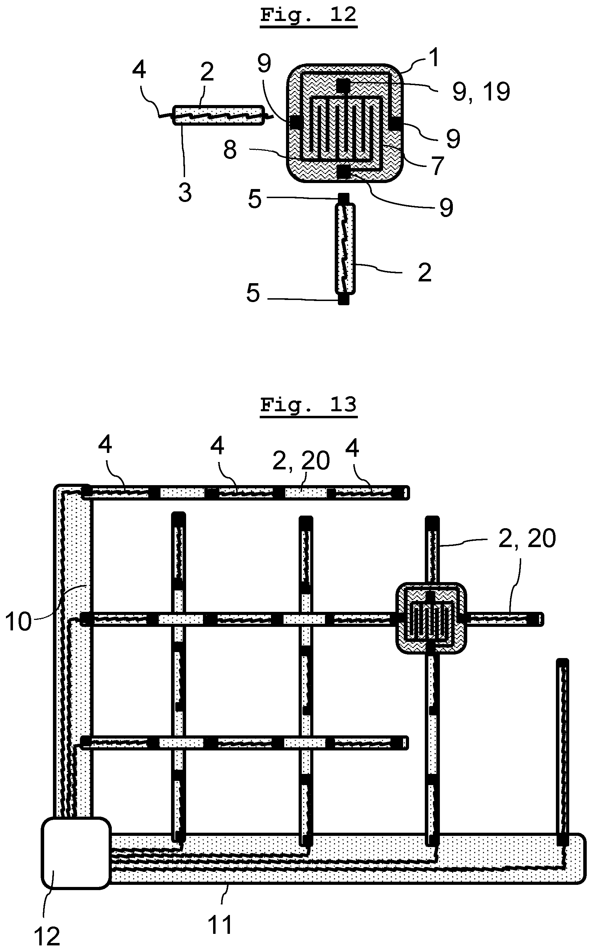

In Fig. 12 ist eine Art Bausatzsystem für Sensornetze dargestellt, umfassend Sensorpunkte 1, welche vier Anschlusspunkte 9 aufweisen und Verbindungselemente 2, welche an beiden Enden Anschlussmöglichkeiten, insbesondere Anschlusspunkte 5, aufweisen. Die vier Anschlusspunkte 9 sind bevorzugt jeweils mittig entlang einer der vier Seitenkante des Sensorpunkts 1 angeordnet. Die Anschlussmöglichkeiten oder Anschlusspunkte 5 der Verbindungselemente 2 können dadurch gebildet sein, dass der Leiter 4 des Verbindungselements 2 frei liegt, oder alternativ ein Anschlusspunkt 5 leitend am Leiter 4 angeschlossen ist (beide Varianten sind veranschaulicht). Durch Anbringen der freien Leiterenden oder Anschlusspunkte 5 der Verbindungselemente 2 an den Anschlusspunkte 9 von Sensorpunkten 1 lassen sich beliebig vielen Sensorpunkte 1 und Verbindungselemente 2 entlang von Reihen und Spalten (erste und zweite Richtung von Gitterlinien, die quer zueinander stehen) zu einem Netz anordnen.

In Fig. 13 ist eine weitere Ausführungsvariante von Verbindungselementen 2 dargestellt, welche sich in Form von Verbindungslinien 20 entlang von ganzen Gitterlinien des Netzes erstrecken. Im Beispiel der Fig. 13 sind an einem Substrat 3 jeder Verbindungslinie 20 mehrere Leiter 4 angeordnet, wobei die Längsrichtung der Leiter 4 in Längsrichtung des Substrats 3 angeordnet ist und zwischen den Leitern 4 Abstände in Längsrichtung vorliegen. An beiden Endbereichen jedes Leiters 4 sind Anschlussmöglichkeiten bzw. Anschlusspunkte 5 vorgesehen. Wie veranschaulicht ist, können Sensorpunkte 1 an Kreuzungspunkten, T-Punkten und Eckpunkten je einer Verbindungslinie 20 einer ersten Richtung und je einer Verbindungslinien 20 einer zweiten Richtung, quer zur ersten, angeordnet werden. Die Kreuzungspunkte der Verbindungslinien 20 hinter den Sensorpunkten 1 sind dabei durch das Substrat 3 gebildet. Das Substrat 3 der Verbindungslinien 20, welches zwischen zwei Anschlusspunkten 9 eines Sensorpunktes 1 vorliegt, kann optional nach Errichten des Netzes entfernt werden.

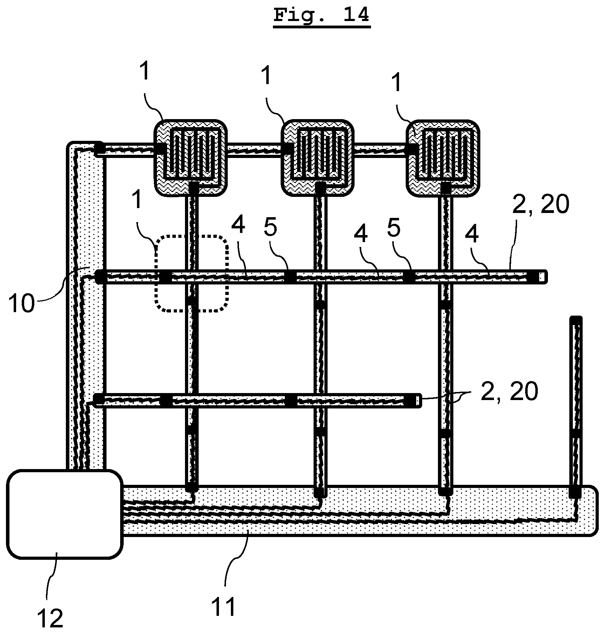

In Fig. 14 ist eine weitere Variante von Verbindungselementen 2 dargestellt, welche sich in Form von Verbindungslinien 20 entlang von ganzen Gitterlinien des Netzes erstrecken. Im Unterschied zu Fig. 9 weisen diese einen durchgehenden Leiter 4 auf, sodass der Stromfluss entlang der jeweiligen Gitterlinie durch den Leiter 4 erfolgt. Entlang der Verbindungslinie 20 sind mit Abstand zueinander Anschlussmöglichkeiten vorhanden, an welchen der Leiter 4 oder ein mit dem Leiter 4 leitend verbundener Anschlusspunkt 5 zur Kontaktierung frei liegt. In diesem Fall ist es ausreichend, wenn der Sensorpunkt 1 nur eine Anschlussmöglichkeit bzw. je einen Anschlusspunkt 9 je Gitterrichtung aufweist. Die Verbindung der Sensorpunkte 1 je einer Gitterlinie erfolgt durch den Leiter 4, welcher hinter oder vor der Fläche des Sensorpunktes 1 verläuft und durch das Substrat 3 gegenüber dem Sensorpunkt 1 elektrisch isoliert ist.

Verbindungselemente 2 in Form von Verbindungslinien 20 eignen sich insbesondere auch für dreidimensionale Sensorpunkte bzw. Sensorpunkte mit Sandwichaufbau, beispielsweise gemäß Fig. 10, wobei die Verbindungslinien 20 der beiden Gitterrichtungen in unterschiedlichen Ebenen vorliegen und somit an gegenüberliegenden Flächen des Sensorpunktes 1 über diesen verlaufen.

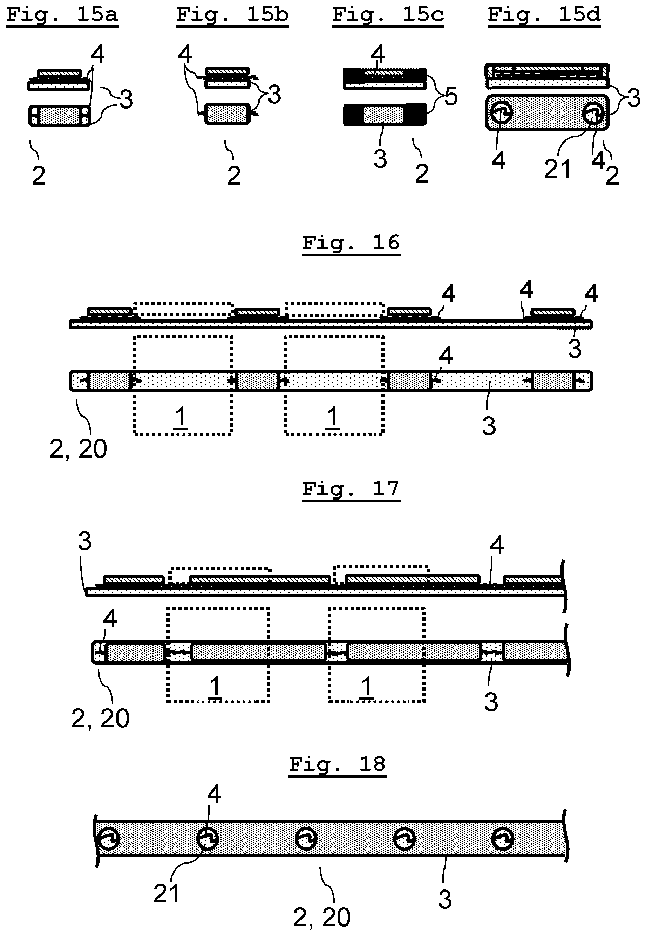

In den Fig. 15a bis 15d sind mögliche Varianten von Verbindungselementen 2 zur Verbindung zweier Sensorpunkte 1 miteinander in Ansicht von vorne und von oben dargestellt. Wie dargestellt kann der Leiter 4 zwischen zwei Lagen des Substrats 3 vorliegen, wobei die beiden Lagen bevorzugt aus demselben Material, insbesondere Elastomer bestehen können. Wie in Fig. 15a dargestellt, kann die erste Lage länger als die zweite Lage ausgeführt sein, wobei der Leiter 4 beidseits der zweiten Lage auf der ersten Lage freiliegt.

Wie in Fig. 15b dargestellt, können die erste Lage und die zweite Lage gleich lang ausgeführt sein, wobei der Leiter 4 beidseits zwischen den Lagen herausragt.

Wie in Fig. 15c dargestellt, kann in Abwandlung der Fig. 11a am freien Ende des Leiters 4 leitendes Material als Anschlusselement bzw. Anschlusspunkt 5 vorliegen.

Wie in Fig. 15d dargestellt, kann der Leiter 4 allseits zwischen zwei Lagen eines Substrats 3 eingeschlossen sein, wobei an jedem Endbereich des Verbindungselements 2 im flächigen Bereich zumindest einer der Lagen eine Öffnung 21 vorliegt.

In Fig. 16 ist eine mögliche Ausführungsvariante einer Verbindungslinie 20 (Ausführungsvariante der Fig. 13) dargestellt, wobei über einem Teilbereich jedes einzelnen Leiters 4 ein Streifen eines isolierenden Materials angebracht ist, wobei beide Enden jedes einzelnen Leiters 4 beidseits des Streifens freiliegen. Die Streifen liegen bevorzugt so wie das durchgehende Substrat 3 der Verbindungslinie 20 als elastomere Folie vor.

In Fig. 17 ist eine mögliche Ausführungsvariante einer Verbindungslinie 20 (Ausführungsvariante der Fig. 14) dargestellt, wobei an mehreren Teilbereichen des einzelnen Leiters 4 je ein Streifen eines isolierenden Materials angebracht ist, wobei die Steifen mit Abstand zueinander in Längsrichtung des Substrats 3 vorliegen und der einzelne Leiter 4 zwischen den Streifen frei liegt. Die Streifen liegen bevorzugt so wie das durchgehende Substrat 3 der Verbindungslinie 20 als elastomere Folie vor.

In Fig. 18 ist eine mögliche Ausführungsvariante einer Verbindungslinie 20 (Ausführungsvariante der Fig. 14) dargestellt, wobei der einzelne Leiter 4 zwischen zwei Lagen des Substrats 3, bzw. zwischen zwei elastomeren Folien vorliegt. Zumindest eine der Lagen ist mit mehreren mit Abstand in Längsrichtung der Verbindungslinie 20 zueinander vorliegenden Öffnungen 21 versehen, wobei der einzelne Leiter 4 im Bereich der Öffnungen 21 frei liegt.

An oder statt den freien Enden oder Abschnitten der Leiter 4 der Fig. 16-18 können Anschlusselemente bzw. Anschlusspunkte 5 vorliegen. Beispielsweise können die Öffnungen 21 mit leitfähigem Material verschlossen oder ausgegossen sein.

Für eine maschinelle Fertigung der Verbindungselemente 2 kann eine Endlosbahn oder ein abgespulter Streifen des Substrats 3 durch eine Maschine transportiert werden, welche einen zweidimensional verformten Leiter 4 entweder durchgehend oder in Abschnitten vereinzelt am Substrat 3 positioniert und wobei eine zweite Lage eines Substrats entweder durchgehend oder in Streifen über dem Leiter 4 positioniert wird. Das zweidimensionale Verformen des Leiters 4 kann dabei in der Maschine erfolgen, oder der Leiter 4 kann der Maschine im zweidimensional verformten Zustand zugeführt werden. Optional kann die Maschine im Bereich der Kontaktstellen des Endlosbandes Anschlusselemente bzw.

Anschlusspunkte 5 anbringen. Das Verbinden der beiden Lagen Substrat kann durch Kleben, Verschweißen oder Verpressen erfolgen.

Am Ausgang der Maschine wird ein endloses bzw. langes Band von Verbindungslinien insbesondere gemäß einer der Figuren 16 bis 18 erhalten, von welchem Endlosband Verbindungslinien 20 in der benötigten Länge oder einzelne Verbindungselemente 2 gemäß den Fig. 15a, 15c und 15d abgeschnitten werden können. Die Varianten der Fig. 15a und Fig. 15b können auch durch Positionieren eines "endlosen" Leiters 4 zwischen zwei "endlosen" Lagen Substrat 3 erhalten werden, wobei nach dem Abschneiden der einzelnen Verbindungselemente 2 zumindest eine (Fig. 15a) oder beide (Fig. 15b) der Lagen des Substrats 3 an beiden Endbereichen des Verbindungselements 2 entfernt wird bzw. werden.

Das Substrat 3 bzw. die Bänder oder Streifen des Substrats 3 liegen in einer Breite vor, welche breiter ist als die Breite der von der zweidimensionalen Form des Leiters eingenommenen Fläche.

In einer anderen Herstellungsvariante kann der zweidimensional verformte Leiter 4 im Substrat 3 eingeschlossen werden, indem dieser auf einer Hälfte eines breiteren Streifens bzw. einer breiteren Bahn eines Substrats 3 platziert wird, deren andere Hälfte um den Leiter 4 gefaltet wird, wobei die freien Enden der beiden Hälften miteinander verbunden, insbesondere verschweißt oder verklebt, werden.

Es ist auch möglich mehrere zweidimensional verformte Leiter 4 parallel und mit Abstand zueinander auf einer breiten Folienbahn oder einem breiten Folienstreifen zu platzieren und mit einer zweiten breiten Folienbahn oder einem zweiten breiten Folienstreifen abzudecken, wobei die Folienbahnen oder Folienstreifen in den Bereichen zwischen den Leitern 4 verklebt oder verschweißt werden. Durch Schneiden der Folienbahnen oder Folienstreifen in Längsrichtung können einzelne Verbindungslinien 20 erhalten werden und durch Schneiden in Querrichtung einzelne Verbindungselemente 2 erhalten werden. Natürlich können auf diese Weise auch Verbindungslinien 20 und Verbindungselemente 2 erhalten werden, welche zwei oder mehr parallele und zueinander beabstandete Leiter 4 enthalten (z.B. für Sammelleitungen 10, 11 oder für Verbindungselemente 2 oder Verbindungslinien 20, welche einen "Messleiter" und einen "Steuerleiter" oder "Busleiter" aufweisen).

## Patentansprüche

1. Zweidimensionales Netz umfassend Sensorpunkte (1), welche die Knotenpunkte des Netzes bilden und längliche Verbindungselemente (2) umfassend ein Substrat (3) und einen Leiter (4), welche die Kanten des Netzes bilden, wobei das Netz in jeder seiner zwei Dimensionen zumindest zwei Kanten aufweist, welche Gitterlinien des Netzes bilden, wobei an jedem Sensorpunkt (1) ein Stromfluss oder eine kapazitive Kopplung von den Leitern (4) einer ersten Gitterlinie zu den Leitern (4) einer zweiten Gitterlinie möglich ist, wobei die erste und zweite Gitterlinie quer zueinander stehen und der Stromfluss oder die kapazitive Kopplung von einer Messgröße des Sensorpunkts (1) abhängig ist, **dadurch gekennzeichnet, dass** das Netz aus Sensorpunkten (1) und Verbindungselementen (2) im Bereich zwischen den Kanten offen ist und die Verbindungselemente (2) jeweils ein dehnbares Substrat (3) aufweisen, welches Substrat (3) als ein gerader Streifen vorliegt, auf welchem zumindest ein Leiter (4) vorliegt, welcher von einem Endbereich des Verbindungselements (2) entlang dessen Längsrichtung zu einem zweiten Endbereich des Verbindungselements (2) verläuft, wobei der Leiter (4) aus einem nicht dehnbaren Material besteht und der Leiter (4) einen gewundenen oder geknickten Verlauf am dehnbaren Substrat (3) aufweist, sodass die einzelnen Abschnitte des Leiters (4) quer zur Längsrichtung des jeweiligen Verbindungselements (2) verlaufen.

2. Netz nach Anspruch 1, **dadurch gekennzeichnet, dass** das Substrat (3) eine elastische Kunststofffolie ist und der Leiter (4) bevorzugt aus Metall besteht.

3. Netz nach einem der Ansprüche 1 bis 2, **dadurch gekennzeichnet, dass** alle Leiter (4), welche entlang einer gemeinsamen Gitterlinie des Netzes vorliegen, entweder direkt leitend verbunden sind oder durch Leiterbahnen (7, 8), welche an den Sensorpunkten (1) vorliegen.

4. Netz nach einem der Ansprüche 1 bis 3, **dadurch gekennzeichnet, dass** an zumindest einigen Sensorpunkten (1) jeweils ein Bauteil in Form einer Diode (15), eines Transistors (16) oder eines adressierbaren Schalters (22) vorliegt, welches Bauteil im Strompfad des Sensorpunktes (1) zwischen den Leitern (4) einer ersten Gitterlinie und den Leitern (4) einer zweiten Gitterlinie angeordnet ist, wobei die erste und zweite Gitterlinie quer zueinander stehen.

5. Netz nach einem der Ansprüche 1 bis 4, **dadurch gekennzeichnet, dass** an zumindest einem Sensorpunkt (1) zwei Leiterbahnen (7, 8) vorliegen, wobei die erste Leiterbahn (7) mit den Leitern (4) einer ersten Gitterlinie des Netzes elektrisch leitend direkt verbunden ist und die zweite Leiterbahn (8) mit den Leitern (4) einer zweiten Gitterline des Netzes elektrisch leitend direkt verbunden ist, wobei die Gitterlinien einander im Bereich des Sensorpunkts (1) kreuzen und wobei zwischen der ersten Leiterbahn (7) und der zweiten Leiterbahn (8) ein für die Messgröße sensitives Material oder Element vorliegt.

6. Netz nach einem der Ansprüche 1 bis 5, **dadurch gekennzeichnet, dass** die Verbindungselemente (2) um das zumindest 1,25-fache, bevorzugt 1,5-fache, insbesondere 2-fache ihrer ungedehnten Länge dehnbar sind.

7. Netz nach einem der Ansprüche 1 bis 6, **dadurch gekennzeichnet, dass** die Länge des Leiters (4) zumindest die 1,5-fache, bevorzugt 2-fache, der geraden Länge des Substrats (3) beträgt, entlang derer er mit gewundenem oder geknicktem Verlauf verläuft.

8. Netz nach einem der Ansprüche 1 bis 7, **dadurch gekennzeichnet, dass** die Leiter (4) je einer Gitterlinie des Netzes mit einem Sammelleiter oder je einem Sammelleiter einer Sammelleitung (10, 11) verbunden sind, welche Sammelleitungen (10, 11) zu einer Auswerteelektronik (12) führen.

9. Verwendung eines Netzes nach einem der Ansprüche 1 bis 8, **dadurch gekennzeichnet, dass** dieses an einem Körper (13) befestigt wird, wobei der Körper (13) bevorzugt ein Gegenstand in Form einer Stützvorrichtung für ein Körperteil eines Lebewesens oder ein Gegenstand in Form eines Kleidungsstücks ist.

10. Verwendung nach Anspruch 9, **dadurch gekennzeichnet, dass** der Körper (13) an Positionen zur Anbringung von Sensorpunkten (1) Markierungen aufweist, wobei zumindest einige der Sensorpunkte (1) unter Dehnung ihrer Verbindungselemente (2) an den Markierungen angebracht werden, wobei bevorzugt aneinander angrenzende Markierungen zumindest den Abstand der ungedehnten Verbindungselemente (2) zueinander aufweisen und wobei zumindest einige aneinander angrenzende Markierungen einen Abstand zueinander aufweisen, welcher größer ist als die Länge der ungedehnten Verbindungselemente (2).

11. Verwendung nach einem der Ansprüche 9 bis 10, **dadurch gekennzeichnet**, für den Körper eines oder mehrere der folgenden Merkmale gilt:
- dass das Netz an einer in zwei Dimensionen gekrümmten Oberfläche des Körpers (13) angebracht wird,
- dass der Körper (13) ein Gegenstand ist, wobei der Gegenstand oder zumindest eine Oberfläche oder innere Schicht des Gegenstands Öffnungen aufweist, welche korrespondierend zu Maschen des Netzes vorliegen,
- dass der Körper (13) im Bereich des Netzes verformbar oder elastisch ist.

12. Verwendung nach einem der Ansprüche 9 bis 11, **dadurch gekennzeichnet, dass** ein digitales Modell des Körpers (13) erstellt wird, welches zumindest die Oberfläche des Körpers (13) und die Position der Sensorpunkte (1) des Sensornetz am Körper (13) umfasst und wobei die Messwerte der einzelnen Sensorpunkte (1) des Sensornetzes durch eine Software mit deren Position im digitalen Modell verknüpft wird.

13. Verwendung nach einem der Ansprüche 9 bis 12, **dadurch gekennzeichnet, dass** der Körper (13) ein Gegenstand ist, wobei in einem ersten Schritt der Gegenstand mit Vertiefungen geschaffen wird oder mit solchen versehen wird, wobei danach am Gegenstand zueinander beabstandete Vertiefungen in Größe der einzelnen Sensorpunkte (1) vorhanden sind, welche die Positionen der Sensorpunkte (1) vorgeben und wobei nachfolgend im zweiten Schritt das Netz am Körper (13) platziert wird, wobei dabei die Sensorpunkte (1) des Netzes in den Vertiefungen des Körpers (13) angeordnet werden, wobei der Körper (13) im ersten Schritt bevorzugt mit zusätzlichen Vertiefungen in Größe der Verbindungselemente (2) geschaffen wird oder mit solchen versehen wird, welche zusätzlichen Vertiefungen für Verbindungselemente (2) die Vertiefungen für Sensorpunkte (1) verbinden und wobei nachfolgend im zweiten Schritt das Netz am Körper (13) platziert wird, wobei dabei die Sensorpunkte (1) des Netzes in den Vertiefungen für Sensorpunkte (1) angeordnet werden und die Verbindungselemente (2) in den zusätzlichen Vertiefungen für Verbindungselemente (2), wobei bevorzugt mehrere Abstände, welche zwischen je zwei Sensorpunkten (1) vorliegen und welche durch die Vertiefungen vorgegeben sind, länger sind als die ungedehnte Länge des jeweiligen Verbindungselements (2), welches zwischen den jeweiligen zwei Sensorpunkten (1) verläuft.

14. Verwendung eines Netzes nach Anspruch 9 **dadurch gekennzeichnet, dass**
- im ersten Schritt ein digitales Modell des Körpers (13) erstellt wird,
- im zweiten Schritt Positionen von Messpunkten im digitalen Modell platziert werden,
- im dritten Schritt das Sensornetz unter individuell vornehmbarer Dehnung der einzelnen Verbindungselemente (2) am realen Körper (13) angebracht wird, wobei die Sensorpunkte (1) am realen Körper an den Positionen der Messpunkte im digitalen Modell angeordnet sind,
- im vierten Schritt zumindest eine Messung der Messwerte an den Sensorpunkten (1) vorgenommen wird.

15. Verwendung eines Netzes nach Anspruch 9 **dadurch gekennzeichnet, dass**
- im ersten Schritt der Körper (13) mit Markierungen für Messpunkte hergestellt wird, oder der Körper (13) mit Markierungen für Messpunkte versehen wird,
- im zweiten Schritt das Sensornetz, unter individuell vornehmbarer Dehnung der einzelnen Verbindungselemente am Körper (13) angebracht wird, wobei die Sensorpunkte (1) am Körper (13) an den Positionen der Markierungen für die Messpunkte angeordnet sind,
- im dritten Schritt zumindest eine Messung der Messwerte an den Sensorpunkten (1) vorgenommen wird.

## Claims

1. A two-dimensional network comprising sensor points (1) forming the nodes of the network and elongated connecting elements (2) comprising a substrate (3) and a conductor (4) forming the edges of the network, the network having in each of its two dimensions at least two edges forming grid lines of the network, wherein at each sensor point (1) a current flow or a capacitive coupling is possible from the conductors (4) of a first grid line to the conductors (4) of a second grid line, the first and second grid lines being transverse to one another and the current flow or the capacitive coupling being dependent on a measured parameter of the sensor point (1), **characterized in that** the network of sensor points (1) and connecting elements (2) being open in the area between the edges and the connecting elements (2) each have an extendable substrate (3), which substrate (3) is present as a straight strip, on which at least one conductor (4) is present, which runs from one end region of the connecting element (2) along its longitudinal direction to a second end region of the connecting element (2), the conductor (4) consisting of a non-stretchable material and the conductor (4) having a wound or bent course on the stretchable substrate (3), so that the individual sections of the conductor (4) run transversely with respect to the longitudinal direction of the respective connecting element (2).

2. Network according to claim 1, wherein the substrate (3) is an elastic plastic film and the conductor (4) is preferably made of metal.

3. Network according to one of the claims 1 to 2, wherein all conductors (4), which are present along a common grid line of the network, are either directly conductively connected or by conductor tracks (7, 8), which are present at the sensor points (1).

4. Network according to one of claims 1 to 3, wherein at least at some sensor points (1) there is a component in the form of a diode (15), a transistor (16) or an addressable switch (22), which component is arranged in the current path of the sensor point (1) between the conductors (4) of a first grid line and the conductors (4) of a second grid line, the first and second grid lines being transverse to one another.

5. Network according to one of claims 1 to 4, wherein two conductor tracks (7, 8) are present at least at one sensor point (1), the first conductor track (7) being electrically connected to the conductors (4) of a first grid line of the network and the second conductor track (8) is directly connected in an electrically conductive manner to the conductors (4) of a second grid line of the grid, the grid lines crossing one another in the region of the sensor point (1) and a material or element which is sensitive to the measured variable being present between the first conductor track (7) and the second conductor track (8).

6. A network according to any one of claims 1 to 5, wherein the connecting elements (2) are stretchable by at least 1.25 times, preferably 1.5 times, in particular 2 times their unstretched length.

7. A network according to any one of claims 1 to 6, wherein the length of the conductor (4) is at least 1.5 times, preferably 2 times, the straight length of the substrate (3) along which it runs with a winding or zigzagging course.

8. Network according to one of the claims 1 to 7, wherein the conductors (4) of each of the respective grid lines of the network are connected to a common collection conductor or to a respective collection conductor of a collection line (10, 11), which collection lines (10, 11) lead to an electronic evaluation unit (12).

9. Use of a network according to any one of claims 1 to 8, wherein it is attached to a body (13),
wherein the body (13) is preferably an object in the form of a support device for a body part of a living being or an object in the form of a garment.

10. Use according to claim 9, wherein the body (13) has markings at positions for attaching sensor points (1), wherein at least some of the sensor points (1) are attached to the markings while stretching their connecting elements (2), wherein preferably adjacent markings are spaced at least the distance of the unstretched connecting elements (2) from each other and wherein at least some adjacent markings have a distance from each other which is greater than the length of the unstretched connecting elements (2).

11. Use according to any one of claims 9 to 10, wherein one or more of the following features apply to the body:
- the network is attached to a surface of the body (13) that is curved in two dimensions,
- the body (13) is an object, the object or at least one surface or inner layer of the object having openings corresponding to the meshes of the network
- the body (13) is deformable or elastic in the region of the network.

12. Use according to any one of claims 9 to 11, wherein a digital model of the body (13) is created, which comprises at least the surface of the body (13) and the position of the sensor points (1) of the sensor network on the body (13), and wherein the measured values of the individual sensor points (1) of the sensor network are linked by software to their position in the digital model.

13. Use according to any one of claims 9 to 12, wherein the body (13) is an object, where in a first step the object is created or provided with recesses, wherein thereafter on the object there are recesses in the size of the individual sensor points (1) and that are spaced apart from one another , which recesses define the positions of the sensor points (1), and wherein subsequently in the second step the network is placed on the body (13), wherein the sensor points (1) of the network are thereby arranged in the recesses of the body (13), wherein in the first step the body (13) is preferably created or provided with additional recesses in the size of the connecting elements (2), which additional recesses for connecting elements (2) connect the recesses for sensor points (1), and subsequently in the second step the network is placed on the body (13), the sensor points (1) of the net being placed in the recesses for sensor points (1) and the connecting elements (2) being placed in the additional recesses for connecting elements (2), wherein preferably a plurality of distances which are present between respective two sensor points (1) and which are predetermined by the recesses are longer than the unstretched length of the respective connecting element (2) which runs between the respective two sensor points (1).

14. Use of a network according to claim 9, wherein
- a digital model of the body (13) is created in a first step,
- in a second step positions of measuring points are placed in the digital model,
- in a third step, the sensor network is attached to the real body (13) while individually stretching the individual connection elements (2), whereby the sensor points (1) are arranged on the real body at the positions of the measuring points in the digital model,
- in a fourth step, at least one measurement of measuring values is carried out at the sensor points (1).

15. Use of a network according to claim 9, wherein
- in a first step, the body (13) is manufactured with markings for measuring points, or the body (13) is provided with markings for measuring points,
- in a second step, the sensor network is attached to the body (13), with stretching of the individual connecting elements, the sensor points (1) being arranged on the body (13) at the positions of the markings for the measuring points,
- in a third step, at least one measurement of measuring values is carried out at the sensor points (1).

## Revendications

1. Réseau bidimensionnel comprenant des nœuds détecteurs (1) qui forment les nœuds du réseau et des éléments de liaison allongés (2) comprenant un substrat (3) et un conducteur (4) qui forment les arêtes du réseau, le réseau présentant dans chacune de ses deux dimensions au moins deux arêtes qui forment des lignes de grille du réseau, un flux de courant ou un couplage capacitif étant possible à chaque nœud détecteur (1) depuis les conducteurs (4) d'une première ligne de grille vers les conducteurs (4) d'une deuxième ligne de grille, les lignes de grille étant transversales l'une par rapport à l'autre et le flux de courant ou le couplage capacitif dépendant d'une grandeur à mesurer du nœud détecteur (1), **caractérisé en ce que** le réseau de nœuds détecteurs (1) et d'éléments de liaison (2) est ouvert dans la zone entre les arêtes et les éléments de liaison (2) présentent chacun un substrat extensible (3), ledit substrat (3) se présentant sous la forme d'une bande droite sur laquelle se trouve au moins un conducteur (4) qui s'étend depuis une zone d'extrémité de l'élément de liaison (2) le long de sa longueur jusqu'à une deuxième zone d'extrémité de l'élément de liaison (2), ledit conducteur (4) étant constitué d'un matériau non extensible et le conducteur (4) présentant une allure sinueuse ou pliée sur le substrat extensible (3), de sorte que les différentes parties du conducteur (4) s'étendent transversalement à la direction longitudinale de l'élément de liaison respectif (2).

2. Réseau selon la revendication 1, **caractérisé en ce que** le substrat (3) est un film plastique élastique et le conducteur (4) est de préférence en métal.

3. Réseau selon l'une des revendications 1 à 2, **caractérisé en ce que** tous les conducteurs (4) qui sont présents le long d'une même ligne de grille du réseau sont reliés de manière conductrice soit directement, soit par des pistes conductrices (7, 8) qui sont présentes aux nœuds détecteurs (1).

4. Réseau selon l'une des revendications 1 à 3, **caractérisé en ce qu'**un composant sous la forme d'une diode (15), d'un transistor (16) ou d'un commutateur adressable (22) est présent à au moins quelques nœuds détecteurs (1), ledit composant étant disposé dans le trajet du courant du nœud détecteur (1) entre les conducteurs (4) d'une première ligne de grille et les conducteurs (4) d'une deuxième ligne de grille, la première et la deuxième ligne de grille étant transversales l'une par rapport à l'autre.

5. Réseau selon l'une des revendications 1 à 4 **caractérisé en ce que** deux pistes conductrices (7, 8) sont présentes en au moins un nœud détecteur (1), la première piste conductrice (7) étant directement reliée de manière électriquement conductrice aux conducteurs (4) d'une première ligne de grille du réseau et la deuxième piste conductrice (8) étant directement reliée de manière électriquement conductrice aux conducteurs (4) d'une deuxième ligne de grille du réseau, les lignes de grille se croisant l'une l'autre au nœud détecteur (1) et un élément ou un matériau sensible à la grandeur à mesurer étant présent entre la première piste conductrice (7) et la deuxième piste conductrice (8).

6. Réseau selon l'une des revendications 1 à 5, **caractérisé en ce que** les éléments de liaison (2) sont extensibles d'au moins 1,25 fois, de préférence 1,5 fois, en particulier 2 fois leur longueur non étirée.

7. Réseau selon l'une des revendications 1 à 6, **caractérisé en ce que** la longueur du conducteur (4) est au moins égale à 1,5 fois, de préférence à 2 fois, la longueur rectiligne du substrat (3) le long de laquelle ledit conducteur s'étend avec une allure sinueuse ou pliée.

8. Réseau selon l'une des revendications 1 à 7, **caractérisé en ce que** les conducteurs (4) de chaque ligne de grille du réseau sont reliés à un conducteur collecteur ou à un conducteur collecteur d'une ligne collectrice (10, 11), lesdites lignes collectrices (10, 11) menant à un système électronique d'évaluation (12).

9. Utilisation d'un réseau selon l'une des revendications 1 à 8, **caractérisée en ce que** celui-ci est fixé à un corps (13), le corps (13) étant de préférence un objet sous la forme d'un dispositif de support pour une partie du corps d'un être vivant ou un objet sous la forme d'un vêtement.

10. Utilisation selon la revendication 9, **caractérisée en ce que** le corps (13) présente des marques à des positions pour la fixation de nœuds détecteurs (1), au moins certains des nœuds détecteurs (1) étant fixés aux marques en étirant leurs éléments de liaison (2), de préférence des marques adjacentes ayant au moins le même écartement les unes par rapport aux autres que les éléments de liaison non étirés (2) et au moins certaines marques adjacentes ayant un écartement les unes par rapport aux autres qui est supérieur à la longueur des éléments de liaison non étirés (2).

11. Utilisation selon l'une des revendications 9 à 10, **caractérisée en ce qu'**une ou plusieurs des caractéristiques suivantes s'appliquent audit corps :
- que le réseau est appliqué sur une surface du corps (13) courbe dans deux dimensions,
- que le corps (13) est un objet, l'objet ou au moins une surface ou une couche intérieure de l'objet présentant des ouvertures qui sont présentes en correspondance avec des mailles du réseau,
- que le corps (13) est déformable ou élastique dans la zone du réseau.

12. Utilisation selon l'une des revendications 9 à 11, **caractérisée en ce qu'**un modèle numérique du corps (13) est créé, qui comprend au moins la surface du corps (13) et la position des nœuds détecteurs (1) du réseau de capteurs sur le corps (13), les valeurs de mesure des différents nœuds détecteurs (1) du réseau de capteurs étant associées par un logiciel à leur position dans le modèle numérique.

13. Utilisation selon l'une des revendications 9 à 12, **caractérisée en ce que** le corps (13) est un objet, l'objet, dans une première étape, étant créé avec des cavités ou pourvu de celles-ci, ensuite, sur l'objet, des cavités de la taille des nœuds détecteurs (1) individuels étant prévues de manière espacée les unes des autres, lesdites cavités définissent les positions des nœuds détecteurs (1), et ensuite, au cours de la deuxième étape, le réseau étant placé sur le corps (13), les nœuds détecteurs (1) du réseau étant alors disposés dans les cavités du corps (13), le corps (13) étant de préférence crée avec des cavités supplémentaires de la taille des éléments de liaison (2) ou pourvu avec celles-ci au cours de la première étape, lesdites cavités supplémentaires pour les éléments de liaison (2) reliant les cavités pour les nœuds détecteurs (1) et, ensuite, dans la deuxième étape, le réseau étant placé sur le corps (13), les nœuds détecteurs (1) du réseau étant placés dans les cavités pour les nœuds détecteurs (1) et les éléments de liaison (2) étant placés dans les cavités supplémentaires pour les éléments de liaison (2), plusieurs écartements, qui sont présents entre deux nœuds détecteurs (1) et qui sont prédéfinis par les cavités, étant de préférence plus longues que la longueur non étirée de l'élément de liaison (2) respectif, qui s'étend entre les deux nœuds détecteurs (1) respectifs.

14. Utilisation d'un réseau selon la revendication 9, **caractérisée en ce que**,
- dans une première étape, un modèle numérique du corps (13) est créé,
- dans une deuxième étape, les positions de points de mesure sont placées dans le modèle numérique,
- dans une troisième étape, le réseau de capteurs est appliqué sur le corps réel (13) en étirant les éléments de liaison, ce qui peut être effectué individuellement, les nœuds détecteurs (1) sur le corps réel étant disposés aux positions des points de mesure dans le modèle numérique,
- dans une quatrième étape, au moins une mesure des valeurs mesurées est effectuée aux nœuds détecteurs (1).

15. Utilisation d'un réseau selon la revendication 9, **caractérisée en ce que**,
- dans la première étape, le corps (13) est créé avec des marques pour des points de mesure, ou le corps (13) est pourvu de marques pour des points de mesure,
- dans la deuxième étape, le réseau de capteurs est appliqué sur le corps réel (13) en étirant les éléments de liaison, ce qui peut être effectué individuellement, les nœuds détecteurs (1) sur le corps (13) étant disposés aux positions des marques pour les points de mesure,
- dans la troisième étape, au moins une mesure des valeurs mesurées est effectuée aux nœuds détecteurs (1).
